(19) Europäisches Patentamt
European Patent Office
Office européen des brevets

(11) **EP 0 982 402 A1**

(12) **EUROPEAN PATENT APPLICATION**

(43) Date of publication:
01.03.2000 Bulletin 2000/09

(51) Int. Cl.$^7$: **C12N 15/40**, C07K 14/08, C12N 7/01, A61K 39/12

(21) Application number: 98202737.7

(22) Date of filing: **14.08.1998**

(84) Designated Contracting States:
**AT BE CH CY DE DK ES FI FR GB GR IE IT LI LU MC NL PT SE**
Designated Extension States:
**AL LT LV MK RO SI**

(71) Applicant:
**Stichting Instituut voor Dierhouderij en Diergezondheid (ID-DLO)
8219 PH Lelystad (NL)**

(72) Inventor:
**The designation of the inventor has not yet been filed**

(74) Representative:
**Smulders, Theodorus A.H.J., Ir. et al
Vereenigde Octrooibureaux
Nieuwe Parklaan 97
2587 BN 's-Gravenhage (NL)**

Remarks:
The application is published incomplete as filed (Article 93 (2) EPC).

(54) **Pestivirus vaccination**

(57) The invention relates to the field of eradication or control of viral diseases of animals, more specifically to vaccines used in the eradication or control of pestivirus infections in pigs or ruminants. The invention provides a recombinant nucleic acid derived from a genotype of a pestivirus, and chimeric virus and vaccine derived thereof, wherein a first fragment or part thereof encoding at least one immunodominant part at least partly responsible for providing protection against a wild-type pestivirus infection of a viral protein has been modified to render said immunodominant part incapable of eliciting antibodies specific for said genotype.

## SEQUENCE LISTING

(1) GENERAL INFORMATION:

(i) APPLICANT:
(A) NAME: Stichting Instituut voor Dierhouderij en Diergezondheid
(B) STREET: Edelhertweg 15
(C) CITY: Lelystad
(D) STATE: Flevoland
(E) COUNTRY: the Netherlands
(F) POSTAL CODE (ZIP): 8219 PH

(ii) TITLE OF INVENTION: Pestivirus vaccination

(iii) NUMBER OF SEQUENCES  17

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE. PatentIn Release #1.0. Version #1.30 (EPO)

(v) CURRENT APPLICATION DATA:
APPLICATION NUMBER: EP 98202737.7

(2) INFORMATION FOR SEQ ID NO: 1:

(i) SEQUENCE CHARACTERISTICS.
(A) LENGTH: 32 base pairs
(B) TYPE. nucleic acid
(C) STRANDEDNESS: unknown
(D) TOPOLOGY: unknown

(ii) MOLECULE TYPE: other nucleic acid

(iii) HYPOTHETICAL. NO

(xi) SEQUENCE DESCRIPTION: SEQ ID NO 1:

CCCGGGATCC AAAAAGCCCT GTTGGCWTGG GC                32

(2) INFORMATION FOR SEQ ID NO: 2.

(i) SEQUENCE CHARACTERISTICS:
(A) LENGTH: 22 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 2

GGGGTGCAGT TGTTTGTATC CA                                22

(2) INFORMATION FOR SEQ ID NO: 3

    (i) SEQUENCE CHARACTERISTICS:
      (A) LENGTH: 20 base pairs
      (B) TYPE: nucleic acid
      (C) STRANDEDNESS: unknown
      (D) TOPOLOGY: unknown

    (ii) MOLECULE TYPE: other nucleic acid

    (iii) HYPOTHETICAL: NO

(xi) SEQUENCE DESCRIPTION: SEQ ID NO: 3

CCGAGAACAT CACCCAGTGG                                20

(2) INFORMATION FOR SEQ ID NO: 4:

    (i) SEQUENCE CHARACTERISTICS
      (A) LENGTH: 20 base pairs
      (B) TYPE: nucleic acid
      (C) STRANDEDNESS: unknown
      (D) TOPOLOGY: unknown

    (ii) MOLECULE TYPE: other nucleic acid

    (iii) HYPOTHETICAL: NO

(xi) SEQUENCE DESCRIPTION: SEQ ID NO: 4:

CATGTGCTCC AAACCATGAT                                20

(2) INFORMATION FOR SEQ ID NO: 5:

    (i) SEQUENCE CHARACTERISTICS.
      (A) LENGTH: 227 amino acids
      (B) TYPE: amino acid
      (C) STRANDEDNESS: unknown
      (D) TOPOLOGY: unknown

    (ii) MOLECULE TYPE: protein

    (iii) HYPOTHETICAL: NO

(xi) SEQUENCE DESCRIPTION: SEQ ID NO. 5:

```
Glu Asn Ile Thr Gln Trp Asn Leu Ser Asp Asn Gly Thr Asn Gly Ile
1               5              10             15

Gln His Ala Met Tyr Leu Arg Gly Val Asn Arg Ser Leu His Gly Ile
        20             25             30

Trp Pro Glu Lys Ile Cys Lys Gly Val Pro Thr His Leu Ala Thr Asp
        35             40             45

Val Glu Leu Lys Glu Ile Gln Gly Met Met Asp Ala Ser Glu Lys Thr
        50             55             60

Asn Tyr Thr Cys Cys Arg Leu Gln Arg His Glu Trp Asn Lys His Gly
        65             70             75             80

Trp Cys Asn Trp Tyr Asn Ile Glu Pro Trp Ile Leu Val Met Asn Arg
        85             90             95

Thr Gln Ala Asn Leu Thr Glu Gly Gln Pro Pro Arg Glu Cys Ala Val
        100            105            110

Thr Cys Arg Tyr Asp Arg Ala Ser Asp Leu Asn Val Val Thr Gln Ala
        115            120            125

Arg Asp Ser Pro Thr Pro Leu Thr Gly Cys Lys Lys Gly Lys Asn Phe
        130            135            140

Ser Phe Ala Gly Ile Leu Met Arg Gly Pro Cys Asn Phe Glu Ile Ala
        145            150            155            160

Ala Ser Asp Val Leu Phe Lys Glu His Glu Arg Ile Ser Met Phe Gln
        165            170            175

Asp Thr Thr Leu Tyr Leu Val Asp Gly Leu Thr Asn Ser Leu Glu Gly
        180            185            190

Ala Arg Gln Gly Thr Ala Lys Leu Thr Thr Trp Leu Gly Lys Gln Leu
        195            200            205

Gly Ile Leu Gly Lys Lys Leu Glu Asn Lys Ser Lys Thr Trp Phe Gly
        210            215            220

Ala Tyr Ala
225
```

(2) INFORMATION FOR SEQ ID NO: 6

(i) SEQUENCE CHARACTERISTICS:
  (A) LENGTH: 228 amino acids
  (B) TYPE: amino acid
  (C) STRANDEDNESS: unknown
  (D) TOPOLOGY: unknown

(ii) MOLECULE TYPE: protein

(iii) HYPOTHETICAL: NO

(xi) SEQUENCE DESCRIPTION: SEQ ID NO: 6:

Glu Asn Ile Thr Gln Trp Asn Leu Gln Asp Asn Gly Thr Glu Gly Ile
1               5               10              15

Gln Arg Ala Met Phe Gln Arg Gly Val Asn Arg Ser Leu His Gly Ile
            20              25              30

Trp Pro Glu Lys Ile Cys Thr Gly Val Pro Ser His Leu Ala Thr Asp
            35              40              45

Thr Glu Leu Lys Ala Ile His Gly Met Met Asp Ala Ser Glu Lys Thr
            50              55              60

Asn Tyr Thr Cys Cys Arg Leu Gln Arg His Glu Trp Asn Lys His Gly
65              70              75              80

Trp Cys Asn Trp Tyr Asn Ile Glu Pro Trp Ile Val Leu Met Asn Lys
            85              90              95

Thr Gln Ala Asn Leu Ala Glu Gly Gln Pro Pro Arg Glu Cys Ala Val
            100             105             110

Thr Cys Arg Tyr Asp Arg Asp Ser Asp Leu Asn Val Val Thr Gln Ala
            115             120             125

Arg Asn Ser Pro Thr Pro Leu Thr Gly Cys Lys Lys Gly Lys Asn Phe
            130             135             140

Ser Phe Ala Gly Val Leu Val Gln Gly Pro Cys Asn Phe Glu Ile Ala
145             150             155             160

Val Ser Asp Val Leu Phe Arg Glu His Asp Cys Thr Ser Val Ile Gln
            165             170             175

Gly Thr Ala His Tyr Leu Val Asp Gly Met Thr Asn Ser Leu Glu Ser
            180             185             190

Ala Arg Gln Gly Thr Ala Lys Leu Thr Thr Trp Leu Gly Arg Gln Leu
            195             200             205

Lys Lys Leu Gly Lys Lys Leu Glu Asn Lys Ser Lys Thr Trp Phe Gly
            210             215             220

Ala Tyr Ala Ala
225

(2) INFORMATION FOR SEQ ID NO. 7:

(i) SEQUENCE CHARACTERISTICS.
    (A) LENGTH: 228 amino acids
    (B) TYPE: amino acid

(C) STRANDEDNESS  unknown
(D) TOPOLOGY· unknown

(ii) MOLECULE TYPE: protein

(iii) HYPOTHETICAL: NO

(xi) SEQUENCE DESCRIPTION: SEQ ID NO: 7·

Glu Asn Ile Thr Gln Trp Asn Ser Gln Asp Asn Gly Thr His Gly Ile
1               5               10              15

Gln His Ala Met Phe Gln Arg Gly Val Asn Arg Ser Leu His Gly Ile
        20              25              30

Trp Pro Glu Lys Ile Cys Thr Gly Val Pro Thr His Leu Ala Thr Asp
        35              40              45

Thr Glu Leu Arg Gly Ile Gln Gly Met Met Asp Ala Ser Glu Lys Thr
        50              55              60

Asn Tyr Thr Cys Cys Arg Leu Gln Arg His Glu Trp Asn Lys His Gly
65              70              75              80

Trp Cys Asn Trp Tyr Asn Ile Asn Pro Trp Ile Trp Val Met Asn Lys
            85              90              95

Thr Gln Ala Asn Leu Thr Glu Gly Pro Pro Glu Lys Glu Cys Thr Val
            100             105             110

Thr Cys Arg Phe Asp Lys Glu Ser Asp Met Asn Val Val Thr Gln Ala
        115             120             125

Arg Asp Arg Pro Thr Thr Leu Thr Gly Cys Lys Lys Gly Lys Lys Phe
        130             135             140

Ser Phe Ala Gly Met Ile Ile Glu Gly Pro Cys Asn Phe Asn Val Ser
145             150             155             160

Val Glu Asp Ile Leu Phe Gly Asp Ser Glu Cys Ser Ser Leu Phe Gln
            165             170             175

Asp Thr Ala Leu Tyr Val Val Asp Gly Val Thr Asn Thr Val Glu Ser
        180             185             190

Ala Arg Gln Gly Ala Ala Lys Leu Thr Ser Trp Leu Gly Lys Gln Leu
        195             200             205

Gly Ile Met Gly Lys Lys Leu Glu His Lys Ser Lys Thr Trp Phe Gly
        210             215             220

Ala Asn Ala Ala
225

(2) INFORMATION FOR SEQ ID NO: 8:

(i) SEQUENCE CHARACTERISTICS
    (A) LENGTH: 228 amino acids
    (B) TYPE: amino acid
    (C) STRANDEDNESS: unknown
    (D) TOPOLOGY: unknown

(ii) MOLECULE TYPE: protein

(iii) HYPOTHETICAL: NO

(xi) SEQUENCE DESCRIPTION: SEQ ID NO: 8:

Glu Asn Ile Thr Met Trp Asn Leu Gln Asp Asn Gly Thr Glu Gly Ile
1       5       10       15

Gln Gln Ala Met Phe Leu Arg Gly Val Asn Arg Ser Leu His Gly Ile
      20       25       30

Trp Pro Glu Lys Ile Cys Thr Gly Val Pro Thr His Leu Ala Thr Asp
      35       40       45

Tyr Glu Leu Arg Glu Ile Val Gly Met Met Asp Ala Ser Glu Lys Thr
      50       55       60

Asn Tyr Thr Cys Cys Arg Leu Gln Arg His Glu Trp Asn Lys His Gly
65       70       75       80

Trp Cys Asn Trp Phe His Ile Glu Pro Trp Ile Trp Leu Met Asn Lys
      85       90       95

Thr Gln Asn Asn Leu Thr Glu Gly Gln Pro Pro Lys Glu Cys Ala Val
      100      105      110

Thr Cys Arg Tyr Asp Lys Glu Ala Glu Leu Asn Ile Val Thr Gln Ala
      115      120      125

Arg Asp Arg Pro Thr Thr Leu Thr Gly Cys Lys Lys Gly Lys Asn Phe
      130      135      140

Ser Phe Ala Gly Val Ile Leu Asp Gly Pro Cys Asn Phe Lys Val Ser
145      150      155      160

Val Glu Asp Val Leu Phe Lys Glu His Asp Cys Gly Asn Met Leu Gln
      165      170      175

Glu Thr Ala Ile Gln Leu Leu Asp Gly Ala Thr Asn Thr Ile Glu Gly
      180      185      190

Ala Arg Val Gly Thr Ala Lys Leu Thr Thr Trp Leu Gly Lys Gln Leu
      195      200      205

Gly Ile Leu Gly Lys Lys Leu Glu Asn Lys Thr Lys Ala Trp Phe Gly
      210      215      220

Ala His Ala Ala
225

(2) INFORMATION FOR SEQ ID NO: 9:

(i) SEQUENCE CHARACTERISTICS:
(A) LENGTH: 228 amino acids
(B) TYPE: amino acid
(C) STRANDEDNESS: unknown
(D) TOPOLOGY: unknown

(ii) MOLECULE TYPE: protein

(iii) HYPOTHETICAL: NO

(xi) SEQUENCE DESCRIPTION: SEQ ID NO 9:

Glu Asn Ile Thr Gln Trp Asn Leu Met Asp Asn Gly Thr Glu Gly Ile
1               5               10              15

Gln Arg Ala Met Phe Leu Arg Gly Val Asn Arg Ser Leu His Gly Ile
        20              25              30

Trp Pro Glu Lys Ile Cys Thr Gly Ile Pro Thr His Leu Ala Thr Asp
        35              40              45

Tyr Glu Leu Lys Glu Ile Val Gly Met Met Asp Ala Ser Glu Lys Thr
        50              55              60

Asn Tyr Thr Cys Cys Arg Leu Gln Arg His Glu Trp Asn Lys His Gly
65              70              75              80

Trp Cys Asn Trp Phe His Ile Glu Pro Trp Ile Trp Leu Met Asn Lys
        85              90              95

Thr Gln Ser Asn Leu Thr Glu Gly Gln Pro Leu Arg Glu Cys Ala Val
        100             105             110

Thr Cys Arg Tyr Asp Arg Glu Thr Glu Leu Asn Ile Val Thr Gln Ala
        115             120             125

Arg Asp Arg Pro Thr Thr Leu Thr Gly Cys Lys Lys Gly Lys Asn Phe
        130             135             140

Ser Phe Ala Gly Ile Val Leu Asn Gly Pro Cys Asn Phe Lys Val Ser
145             150             155             160

Val Glu Glu Val Leu Phe Lys Glu His Asp Cys Gly Asn Met Leu Gln
        165             170             175

Glu Thr Ala Ile Gln Leu Leu Asp Gly Ala Thr Asn Thr Ile Glu Gly
        180             185             190

Ala Arg Val Gly Thr Ala Lys Leu Thr Thr Trp Leu Gly Lys Gln Leu
        195             200             205

26

Arg Ile Leu Gly Arg Lys Leu Glu Asn Lys Ser Lys Ser Trp Phe Gly
210            215            220

Ala His Ala Ala
225

(2) INFORMATION FOR SEQ ID NO: 10

(i) SEQUENCE CHARACTERISTICS:
    (A) LENGTH: 228 amino acids
    (B) TYPE: amino acid
    (C) STRANDEDNESS: unknown
    (D) TOPOLOGY: unknown

(ii) MOLECULE TYPE: protein

(iii) HYPOTHETICAL: NO

(xi) SEQUENCE DESCRIPTION: SEQ ID NO: 10:

Glu Asn Ile Thr Gln Trp Asn Leu Gln Asp Asn Gly Thr Glu Gly Ile
1           5           10           15

Gln Arg Ala Met Phe Gln Arg Gly Val Asn Arg Ser Leu His Gly Ile
        20           25           30

Trp Pro Glu Lys Ile Cys Thr Gly Val Pro Ser His Leu Ala Thr Asp
        35           40           45

Ile Glu Leu Lys Thr Ile His Gly Met Met Asp Ala Ser Glu Gly Thr
        50           55           60

Asn Tyr Thr Cys Cys Lys Leu Gln Arg His Glu Trp Asn Lys His Gly
65           70           75           80

Trp Cys Asn Trp His Asn Ile Asp Pro Trp Ile Gln Leu Met Asn Arg
            85           90           95

Thr Gln Ala Asp Leu Ala Glu Gly Pro Pro Val Lys Glu Cys Ala Val
            100           105           110

Thr Cys Arg Tyr Asp Lys Asp Ala Asp Ile Asn Val Val Thr Gln Ala
            115           120           125

Arg Asn Arg Pro Thr Thr Leu Thr Gly Cys Lys Lys Gly Lys Asn Phe
            130           135           140

Ser Phe Ala Gly Thr Val Ile Glu Ser Pro Cys Asn Phe Asn Val Ser
145           150           155           160

Val Glu Asp Thr Leu Tyr Gly Asp His Glu Cys Gly Ser Leu Leu Gln
            165           170           175

Asp Ala Ala Leu Tyr Leu Val Asp Gly Met Thr Asn Thr Ile Glu Asn

180          185          190

Ala Arg Gln Gly Ala Ala Arg Val Thr Ser Trp Leu Gly Arg Gln Leu
   195          200          205

Arg Thr Ala Gly Lys Arg Leu Glu Gly Arg Ser Lys Thr Trp Phe Gly
   210          215          220

Ala Tyr Ala Ala
225

(2) INFORMATION FOR SEQ ID NO: 11:

   (i) SEQUENCE CHARACTERISTICS
      (A) LENGTH: 177 amino acids
      (B) TYPE: amino acid
      (C) STRANDEDNESS: unknown
      (D) TOPOLOGY: unknown

   (ii) MOLECULE TYPE: protein

   (iii) HYPOTHETICAL: NO

   (xi) SEQUENCE DESCRIPTION: SEQ ID NO: 11:

His Leu Asp Cys Lys Pro Glu Phe Ser Tyr Ala Ile Ala Lys Asp Glu
1          5          10          15

Arg Ile Gly Gln Leu Gly Ala Glu Gly Leu Thr Thr Thr Trp Lys Glu
   20          25          30

Tyr Ser Pro Gly Met Lys Leu Glu Asp Thr Met Val Ile Ala Trp Cys
   35          40          45

Glu Asp Gly Lys Leu Met Tyr Leu Gln Arg Cys Thr Arg Glu Thr Arg
   50          55          60

Tyr Leu Ala Ile Leu His Thr Arg Ala Leu Pro Thr Ser Val Val Phe
65          70 ·          75          80

Lys Lys Leu Phe Asp Gly Arg Lys Gln Glu Asp Val Val Glu Met Asn
   85          90          95

Asp Asn Phe Glu Phe Gly Leu Cys Pro Cys Asp Ala Lys Pro Ile Val
   100          105          110

Arg Gly Lys Phe Asn Thr Thr Leu Leu Asn Gly Pro Ala Phe Gln Met
   115          120          125

Val Cys Pro Ile Gly Trp Thr Gly Thr Val Ser Cys Thr Ser Phe Asn
   130          135          140

Met Asp Thr Leu Ala Thr Thr Val Val Arg Thr Tyr Arg Arg Ser Lys
145          150          155          160

28

Pro Phe Pro His Arg Gln Gly Cys Ile Thr Gln Lys Asn Leu Gly Glu
165          170          175

Asp

(2) INFORMATION FOR SEQ ID NO 12

(i) SEQUENCE CHARACTERISTICS
    (A) LENGTH: 178 amino acids
    (B) TYPE: amino acid
    (C) STRANDEDNESS: unknown
    (D) TOPOLOGY: unknown

(ii) MOLECULE TYPE: protein

(iii) HYPOTHETICAL. NO

(xi) SEQUENCE DESCRIPTION: SEQ ID NO: 12.

Leu Pro Val Cys Lys Pro Gly Phe Tyr Tyr Ala Ile Ala Lys Asn Asn
1         5           10          15

Glu Ile Gly Pro Leu Gly Ala Thr Gly Leu Thr Thr Gln Trp Tyr Glu
        20          25          30

Tyr Ser Asp Gly Met Arg Leu Gln Asp Thr Gly Val Val Val Trp Cys
        35          40          45

Lys Gly Gly Glu Ile Lys Tyr Leu Ile Thr Cys Glu Arg Glu Ala Arg
        50          55          60

Tyr Leu Ala Ile Leu His Thr Arg Ala Leu Pro Thr Ser Val Val Phe
65          70          75          80

Glu Lys Ile Ile Asp Gly Lys Glu Gln Glu Asp Val Val Glu Met Asp
        85          90          95

Asp Asn Phe Glu Leu Gly Leu Cys Pro Cys Asp Ala Lys Pro Leu Val
        100          105          110

Arg Gly Lys Phe Asn Thr Thr Leu Leu Asn Gly Pro Ala Phe Gln Met
        115          120          125

Val Cys Pro Ile Gly Trp Thr Gly Thr Val Ser Leu Cys His Trp Ser
        130          135          140

Asn Lys Asp Thr Leu Ala Met Thr Val Val Arg Thr Tyr Lys Arg His
145          150          155          160

Arg Pro Phe Pro Phe Arg Gln Gly Cys Ile Thr Gln Lys Val Ile Gly
        165          170          175

Gly Asp

(2) INFORMATION FOR SEQ ID NO: 13.

(i) SEQUENCE CHARACTERISTICS:
   (A) LENGTH: 176 amino acids
   (B) TYPE: amino acid
   (C) STRANDEDNESS. unknown
   (D) TOPOLOGY. unknown

(ii) MOLECULE TYPE: protein

(iii) HYPOTHETICAL. NO

(xi) SEQUENCE DESCRIPTION: SEQ ID NO: 13:

Glu Phe Ala Cys Arg Glu Asp His Arg Tyr Ala Leu Ala Lys Thr Lys
1               5               10              15

Glu Ile Gly Pro Leu Gly Ala Glu Ser Leu Thr Thr Thr Trp Thr Asp
        20              25              30

Tyr Gln Arg Ser Phe Asp Leu Asp Asp Gly Ala Val Arg Ala Ser Cys
        35              40              45

Val Gly Gly Tyr Phe Arg Phe Arg Glu His Cys Leu Ile Gly Ser Arg
        50              55              60

Tyr Leu Ala Ser Leu His Gln Arg Ala Leu Pro Thr Ser Val Thr Phe
65              70              75              80

Glu Leu Ile Pro Gly Gly Ser Thr Leu Val Glu Glu Glu Met Gly Asp
        85              90              95

Asp Phe Glu Phe Gly Leu Cys Pro Cys Asp Ser Arg Pro Leu Val Arg
        100             105             110

Gly Lys Tyr Asn Ala Thr Leu Leu Asn Gly Ser Ala Phe Gln Leu Val
        115             120             125

Cys Pro Tyr Glu Trp Thr Gly Arg Val Glu Cys Thr Thr Ile Ser Lys
        130             135             140

Ser Thr Leu Ala Thr Glu Val Val Lys Ile Tyr Lys Arg Thr Lys Arg
145             150             155             160

Phe Arg Ser Gly Leu Ala Val Thr His Thr Thr Ile Tyr Glu Glu Asp
        165             170             175

(2) INFORMATION FOR SEQ ID NO: 14:

(i) SEQUENCE CHARACTERISTICS:
   (A) LENGTH: 175 amino acids
   (B) TYPE: amino acid
   (C) STRANDEDNESS: unknown

(D) TOPOLOGY· unknown

(ii) MOLECULE TYPE. protein

(iii) HYPOTHETICAL. NO

(xi) SEQUENCE DESCRIPTION: SEQ ID NO: 14:

Phe Pro Glu Cys Lys Glu Gly Phe Gln Tyr Ala Ile Ser Lys Asp Arg
1               5               10              15

Lys Met Gly Leu Leu Gly Pro Glu Ser Leu Thr Thr Thr Trp His Arg
        20              25              30

Pro Thr Lys Lys Leu Val Asp Ser Met Val Gln Val Trp Cys Glu Gly
        35              40              45

Lys Asp Leu Lys Ile Leu Lys Thr Cys Pro Lys Glu Glu Arg Tyr Leu
        50              55              60

Val Ala Val His Glu Arg Ala Leu Ser Thr Ser Ala Glu Phe Met Pro
65              70              75              80

Ile Ser Asp Gly Thr Ile Gly Pro Asp Val Ile Asp Met Pro Asp Asp
            85              90              95

Phe Glu Phe Gly Leu Cys Pro Cys Asp Ala Lys Pro Val Ile Lys Gly
            100             105             110

Lys Phe Asn Ala Ser Leu Leu Asn Gly Pro Ala Phe Gln Met Val Cys
        115             120             125

Pro Gln Gly Trp Thr Gly Thr Ile Glu Cys Thr Leu Ala Asn Gln Asp
    130             135             140

Thr Leu Asp Thr Thr Val Val Arg Thr Tyr Arg Arg Thr Thr Pro Phe
    145             150             155             160

Gln Arg Arg Lys Trp Cys Ser Tyr Glu Lys Ile Ile Gly Glu Asp
        165             170             175

(2) INFORMATION FOR SEQ ID NO: 15:

(i) SEQUENCE CHARACTERISTICS.
    (A) LENGTH: 175 amino acids
    (B) TYPE: amino acid
    (C) STRANDEDNESS: unknown
    (D) TOPOLOGY: unknown

(ii) MOLECULE TYPE: protein

(iii) HYPOTHETICAL. NO

(xi) SEQUENCE DESCRIPTION  SEQ ID NO: 15

Phe Pro Glu Cys Lys Glu Gly Phe Gln Tyr Ala Ile Ser Lys Asp Lys
1 5 10 15

Lys Ile Gly Leu Leu Gly Pro Glu Ser Leu Thr Thr Thr Trp His Leu
20 25 30

Pro Thr Lys Lys Leu Val Asp Ser Met Val Gln Val Trp Cys Glu Gly
35 40 45

Lys Asp Leu Lys Ile Leu Lys Thr Cys Thr Arg Glu Glu Arg Tyr Leu
50 55 60

Val Ala Val His Glu Arg Ala Leu Ser Thr Ser Ala Glu Phe Met Gln
65 70 75 80

Ile Ser Asp Gly Lys Leu Gly Pro Ser Val Ile Asp Met Pro Asp Asp
85 90 95

Phe Glu Phe Gly Leu Cys Pro Cys Asp Ser Lys Pro Val Ile Lys Gly
100 105 110

Lys Phe Asn Ala Ser Leu Leu Asn Gly Pro Ala Phe Gln Met Val Cys
115 120 125

Pro Gln Gly Trp Thr Gly Arg Ile Glu Cys Thr Ser Val Asn Gln Asp
130 135 140

Thr Leu Asp Thr Thr Val Val Arg Thr Tyr Arg Arg Thr Thr Pro Phe
145 150 155 160

Gln Arg Arg Arg Trp Cys Val Tyr Glu Lys Met Ile Gly Glu Asp
165 170 175

(2) INFORMATION FOR SEQ ID NO: 16:

    (i) SEQUENCE CHARACTERISTICS.
        (A) LENGTH: 176 amino acids
        (B) TYPE: amino acid
        (C) STRANDEDNESS: unknown
        (D) TOPOLOGY: unknown

    (ii) MOLECULE TYPE. protein

    (iii) HYPOTHETICAL: NO

(xi) SEQUENCE DESCRIPTION: SEQ ID NO: 16:

Arg Leu Ala Cys Lys Glu Asp His Arg Tyr Ala Ile Ser Thr Thr Asn
1 5 10 15

Glu Ile Gly Leu His Gly Ala Glu Gly Leu Thr Thr Thr Trp Lys Glu
20 25 30

Tyr Asn His Asn Leu Gln Leu Asp Asp Gly Thr Val Lys Ala Ile Cys
35　　　　40　　　　45

Met Ala Gly Ser Phe Lys Val Thr Ala Leu Asn Val Val Ser Arg Arg
50　　　　55　　　　60

Tyr Leu Ala Ser Leu His Lys Asp Ala Leu Pro Thr Ser Val Thr Phe
65　　　　70　　　　75　　　　80

Glu Leu Leu Phe Asp Gly Thr Ser Pro Leu Thr Glu Glu Met Gly Asp
85　　　　90　　　　95

Asp Phe Gly Phe Gly Leu Phe Pro Phe Asp Thr Ser Pro Val Val Lys
100　　　　105　　　　110

Gly Lys Phe Asn Thr Thr Leu Leu Asn Gly Ser Ala Phe Tyr Leu Val
115　　　　120　　　　125

Cys Pro Ile Gly Trp Thr Gly Val Ile Glu Cys Thr Ala Val Ser Pro
130　　　　135　　　　140

Thr Thr Leu Arg Thr Glu Val Val Lys Thr Tyr Phe Arg Glu Lys Pro
145　　　　150　　　　155　　　　160

Phe Pro Tyr Arg Arg Asp Cys Val Thr Thr Thr Val Glu Asn Glu Asp
165　　　　170　　　　175


(2) INFORMATION FOR SEQ ID NO: 17:

(i) SEQUENCE CHARACTERISTICS:
(A) LENGTH: 176 amino acids
(B) TYPE: amino acid
(C) STRANDEDNESS: unknown
(D) TOPOLOGY: unknown

(ii) MOLECULE TYPE: protein

(iii) HYPOTHETICAL. NO


(xi) SEQUENCE DESCRIPTION: SEQ ID NO: 17:

Arg Leu Ala Cys Lys Glu Asp Tyr Arg Tyr Ala Ile Ser Thr Thr Asp
1　　　　5　　　　10　　　　15

Glu Ile Gly Leu Leu Gly Ala Gly Gly Leu Thr Thr Thr Trp Lys Glu
20　　　　25　　　　30

Tyr Asn His Asp Leu Gln Leu Asn Asp Gly Thr Val Lys Ala Ser Cys
35　　　　40　　　　45

Val Ala Gly Ser Phe Lys Val Thr Ala Leu Asn Val Val Ser Arg Arg
50　　　　55　　　　60

Tyr Leu Ala Ser Leu His Lys Lys Ala Leu Pro Thr Ser Val Thr Phe
65 70 75 80

Glu Leu Leu Phe Asp Gly Thr Asn Pro Ser Thr Glu Glu Met Gly Asp
85 90 95

Asp Phe Arg Ser Gly Leu Phe Pro Phe Asp Thr Ser Pro Val Val Lys
100 105 110

Gly Lys Tyr Asn Thr Thr Leu Leu Asn Gly Ser Ala Phe Tyr Leu Val
115 120 125

Cys Pro Ile Gly Trp Thr Gly Val Ile Glu Cys Thr Ala Val Ser Pro
130 135 140

Thr Thr Leu Arg Thr Glu Val Val Lys Thr Tyr Phe Arg Asp Lys Pro
145 150 155 160

Phe Pro His Arg Met Asp Cys Val Thr Thr Thr Val Glu Asn Glu Asp
165 170 175

**Description**

The invention relates to the field of eradication or control of viral diseases of animals, more specifically to vaccines used in the eradication or control of pestivirus infections in pigs or ruminants.

Vaccination can serve two purposes: 1) to protect the vaccinated individual against signs of the disease in case of contact with the agent (individual immunity), and 2) to reduce each individual's chance of becoming infected when it is a part of a population of vaccinated individuals (herd immunity) . The development of marker vaccines for veterinary use has led to new concepts for control and eradication of economically important viral diseases of livestock. The use of a marker vaccine allows serological discrimination between vaccinated and field-virus infected animals, and thereby a controlled elimination of the virus.

For instance, in most member states of the EU vaccination against Aujeszky's disease (AD) is allowed with gE-negative (deleted) vaccines only. Animals infected with an AD field virus do develop antibodies against gE. An ELISA test which detects these antibodies is used for the detection of infected animals, which subsequently may be removed from a herd, and for monitoring the status of wild-type Aujeszky's disease virus (ADV) infections in a herd during or after vaccination campaigns. Eventually, the aim is to reach a field or wild-type virus free status of a herd. Vaccination can then be discontinued and a serological surveillance program to guard this status should come into force.

However, said marker technology which is used in the case of ADV, a large DNA virus, simply by deleting fragments from the genome of said virus, can not be used with small RNA viruses, such as *Pestiviruses*, whereby it is impossible to delete fragments from the small RNA genome without disrupting its viability.

The genus *Pestivirus* of the family *Flaviviridae* conventionally consists of classical swine fever virus (CSFV), Border disease virus (BDV), and bovine viral diarrhoea virus (BVDV). Genomes of several BDV and CSFV strains have been sequenced, individual pestiviral proteins have been expressed and infectious copy RNA viruses have been generated (Renard et al., 1987 EP application 0208672; Collett et al., 1988, Virology 165, 191-199; Deng and Brock, 1992, Virology 1991, 865-679; Meyers et al., 1989, Virology 171, 555-567; Moormann et al., 1990, Virology 177, 184-188; Meyers et al., 1989, EP 89104921; Moormann and Wensvoort, 1989, PCT/NL90/00092; Moormann and Van Rijn; 1994, PCT/NL95/00214; Ridpath et al., 1997, Virus Res. 50: 237-243; Becker et al., 1998, J. Virol. 72:5165-5173).

The pestivirus genome is a positive-stranded RNA molecule of about 12.5 kilobases containing one large open reading frame. The open reading frame is translated into a hypothetical polyprotein of approximately 4,000 amino acids, which is processed by virus- and cell-encoded proteases. The open reading frame is flanked by two highly conserved small nontranslated regions, which are probably involved in the replication of the genome. The 5'-noncoding region also plays a role in initiation of translation.

The polyprotein which is co- and posttranslationally processed by cellular and viral proteases contains all the viral structural and nonstructural proteins (for review see C.M. Rice: In Fields Virology, Third Edition, 1996 ) *Flaviviridae*: The Viruses and their Replication: Chapter 30: pp. 931-959. The viral structural proteins, among which the envelope proteins E[rns], E1 and E2, are located in the N-terminal part of the polyprotein. The nonstructural proteins among, which the serine protease NS3 and RNA replicase complex NS5A and NS5B, are located in the C-terminal part of the polyprotein.

To date, BDV and BVDV have been isolated from different species (ruminants and pigs), whereas CSFV seems to be restricted to swine. Pestiviruses are structurally and antigenically closely related.

Envelope glycoprotein E2 is the most immunodominant and most variable protein of pestiviruses. We cloned E2 genes of many different pestivirus strains, including those from a deer and a giraffe. The E2 genes were transiently expressed, characterised with monoclonal antibodies, sequenced and compared, P.A. van Rijn et al., 1997, Virology, 237: 337-348. Based on these data, we can delineate six major genotype groups within the pestivirus genus which each correspond to genotype specific E2 protein. Within groups, the antibodies against E2 strongly cross-react, in ELISA as well as in neutralisation tests, in between genotype groups, E2 proteins are in general antigenically unrelated and E2-sera or E2-specific antibodies against one genotype are not or only little reactive in inter-genotype ELISA or neutralisations tests with any one of the other genotype E2 proteins.

Four genotype groups correspond to the defined traditionally known genotypes CSFV, BVDV and BDV, whereas the two other groups are relatively new genotypes within the pestivirus genus.

One group (genotype CSFV) comprises CSFV strains isolated from swine. A second group (genotype BDV) comprises BDV strains Moredun, L83 and X818, which have been isolated from sheep, and strain F from swine. A third group (genotype BVDV II) comprises strain BD78 from sheep, strain 5250 from swine and strain 178003 from cattle. On the basis of E2, these viruses are very similar to BVDV strains associated with acute severe outbreaks of bovine viral diarrhoea, and comprise the so called type II BVDV. The fourth group (genotype BVDV type I) comprises BVDV strains predominantly originating from cattle. This BVDV-group can be divided into two subtypes or subgroups BVDV-1a and 1b: BVDV-1a comprises viruses from the USA, like NADL and Oregon, and some others, like 150022 and 1138 from Europe. Subgroup BVDV-1b comprises strain Osloss and several Dutch isolates. The fifth (genotype Deer) and sixth (genotype Giraffe) "group" could be proposed as two new genotypes and contain strains Deer and Giraffe, respectively.

In general, antibodies raised against one genotype pestivirus are only of little protective value when tested with other genotype-specific wild-type pestivirus infections, no or only little cross-protection between genotypes exists.

Animals infected with a pestivirus develop detectable antibodies against E$^{rns}$, E2 and NS3. Antibodies directed against E2 are strongly virus neutralising and are generally thought to be the most protective within the genotype tested. Considering the central role antibodies directed against E2 play in protection, most focus has been given at developing marker vaccines comprising E2 or (recombinant) E2 subunit vaccines, all capable of generating protective antibody levels directed against E2 to be used against pestivirus infections. Antibodies directed against E$^{rns}$ in general are genotype specific, have in general a low virus neutralising capacity and are considered having limited but additive protective capacity; those directed against NS3 have no neutralising capacity at all and are thought not to be protective, despite the fact that these are often broadly reactive within and between the several genotype groups.

CSFV is a notifiable disease for which in general the veterinary authorities are responsible for its eradication. Eradication generally occurs by a stamping-out policy, whereby all animals in an infected outbreak area are killed and destroyed, in the hope of thereby destroying the virus and preventing further transmission to other populations.

Authorities go to great length and costs in stamping-out, during the (latest) CSFV-epizootic in the Netherlands in 1997-1998, a estimated total of 9.000.000 pigs (on a total pig population of approximately 15.000.000) were killed and destroyed in relation to the outbreak. Such costs would markedly be reduced when vaccination was used to protect populations at risk, however, current available vaccines are in general not deemed fit for the purpose.

Several arguments against CSFV vaccination using currently available vaccines are used. For example, it is reasoned that vaccination clouds the issues. It complicates the diagnosis of the disease, particularly by serological testing when post-vaccination antibodies cannot be distinguished from post-infection antibodies and thereby masks occurrence of the disease. Furthermore, it is reasoned that vaccination does not protect every animal, some animals may harbour the causal agent, becoming latent carriers and perpetuating the disease. Also, vaccination hampers the free trade in animals, particularly for export purposes when vaccinated animals cannot be identified, restricting income from export from vaccinated regions.

When applied, CSFV vaccination would generally be performed during a mass campaign in an area where an outbreak of CSFV has occurred. This calls for rapid vaccination of large numbers of animals in a relatively short period. In such a mass campaign it is of imminent importance that an adequate protection at the herd level (the number of pigs that are protected against the wild type virus infection) is achieved rapidly. Using whole killed CSFV vaccines has been deserted long ago due to their lack of antigenic mass and thus protection. E2 subunit vaccines in general are difficult to produce in the large quantities needed for mass campaigns. Waiting for several weeks after a first vaccination for a second vaccination, as is required for at least one subunit CSFV vaccine in order to achieve protection, greatly hampers and delays the control of the disease and does not provide the required rapid herd immunity. Using a non-marker vaccine, such as the C-strain virus, does not provide the required serological discrimination whereby wild-type infected animals or populations can be identified and culled. Recombinant ADV-CSFV-E2 marker vaccine, in theory a vaccine carrying many desired characteristics to try to achieve successful vaccination (it is live, its is a marker, it protects) also generates antibodies directed against ADV, which makes it impossible to use where pigs have maternal or otherwise (via vaccination or wild-type infection) derived antibodies directed against ADV. Furthermore, its use in CSFV eradication would frustrate the serological detection and eradication of ADV-infected pigs, and thus in the end be counterproductive as a whole to the pig industry.

In short, current available vaccines against CSFV (reviewed in Moormann et al., 1996, Proc. 14$^{th}$ Intern. Pig. Vet. Society Congress, pp. 25-29, Bologna, Italy), such as the classical vaccine C-strain virus (non-marker), the recombinant ADV-CSFV-E2 marker vaccine, and a E2 sub-unit vaccine are at present all considered to be insufficient by the authorities to reach one or more of above defined goals relating to immunity, therefore a need for a better CSFV vaccine exists.

The same goes for the other pestiviruses such as BVDV or BDV, despite the fact that these do not cause notifiable disease. With BVDV and BDV, which cross-infect between ruminant species and even can cause infections in pigs, live vaccines in general are not sufficiently broadly immunoreactive, and cannot prevent or (to the contrary) even cause vertical transmission from infected cow or sheep to its foetus, thereby enhancing further transmission of the virus. Serological discrimination in case of BVDV is further hampered by the existence of the various serotypes (BVDV1a, BVDV1b, or BVDV2). Whole killed vaccines in general do not have enough antigenic mass to protect at all, or only for a short period, making repeated vaccinations necessary and (experimental) subunit BVDV vaccines suffer from the antigenically narrow spectrum they can cover.

The invention provides a recombinant nucleic acid derived from a genotype of a pestivirus wherein a first fragment or part thereof encoding at least one immunodominant part at least partly responsible for providing protection against a wild-type pestivirus infection of a viral protein has been modified to render said immunodominant part incapable of eliciting antibodies specific for said genotype. Surprisingly, by rendering said immunodominant part less immunogenic and thus less protective, still a protective vaccine can be provided, as demonstrated below, that, however, allows for the serological discrimination needed in control and/or eradication programmes whereby vaccinated animals or (partly) vaccinated populations of animals or tested (and subsequently culled) for the presence of antibodies against the wild-type virus causing the disease under control.

It is than essential that the RNA genome in some way remains its ability to replicate, therefore, the nucleic acid modification, as provided by the invention, eventually allows for generating replicating virus (chimeric virus) comprising most of the original genome, supplemented with a modified part. Such a modification as provided by the invention lives up to the structural requirements of both the encoding RNA and of the encoded proteins, thereby allowing replication of the virus and translation of the genome in structurally relevant proteins with which a viable virus particle can be generated. Such modifications can for example comprise deleting or introducing a codon encoding cysteine, thereby allowing for disrupted or enhanced disulfide bridge formation in between viral proteins, thereby effecting dimerisation or multimerisation. Also, the invention provides deleting small fragments, often only single codons, or at least at several positions, to render the protein with the desired antigenic characteristics.

In a preferred embodiment the invention provides a recombinant nucleic acid derived from a genotype of a pestivirus wherein a first fragment encoding at least one immunodominant part, at least partly responsible for providing protection against a wild-type pestivirus infection, of a pestiviral protein has been replaced by a second fragment encoding an antigenically distantly related or unrelated part of a pestiviral protein from another genotype. Herewith the structural requirements are particularly well met. Constructing or producing a recombinant nucleic acid as provided by the invention in essence comprises two aspects. A first aspect, relating to the selection of a part of a pestiviral protein selected for replacement, a second aspect relating to the part of pestiviral protein of the desired wild-type virus by which serological discrimination is enabled. These two parts are the same; often, an immunodominant protein elicits antibodies by which an infection can be detected, but it is the line of expectations that the same antibodies are at least partly protective, especially when these are genotype specific. For serological discrimination of pestivirus genotypes it does not suffice providing protection with a vaccine comprising the protective proteins E2 or $E^{rns}$ but not the non-protective NS3 protein, and detecting infections with tests based on NS3. NS3 is not genotype specific, at least it does not elicit genotype specific antibodies to allow discrimination between genotypes. Such diagnostic tests can thus hardly be used in the aftermath of vaccination campaigns against for example CSFV in pigs. Circulating NS3-BDV or NS3-BVDV antibodies will cause a plethora of false-positive results, leading to suspicions of CSFV infections when in fact there aren't any in the pig population tested.

In constructing a vaccine, one has to take into account what (type of) serological test is preferred once the vaccine is employed in the field. It should be genotype specific, which blocks using diagnostic tests based on NS3. However, selecting E2 or $E^{rns}$ as diagnostic antigens hampers developing a vaccine which uses the protective properties of these proteins. The invention surprisingly provides vaccines in which specific immunodominant parts, in general thought responsible and necessary for generating protection, have been modified or replaced with antigenically unrelated or only distantly related (for that genotype) parts, thus allowing serological discrimination, without seriously hampering protective properties.

Contrary to what seems common sense in producing a vaccine, especially in the field of pestiviruses where so much focus has been given to the presence of protective E2 protein (of which, in a preferred embodiment of the invention, a protective part is at least partly replaced by an antigenically unrelated part of E2 of another genotype pestivirus), the invention provides pestiviral recombinant nucleic acid which has been deprived from at least part of its (genotype specific) protective capacities to allow for serological detection of wild-type infections with said genotype.

In a preferred embodiment of the invention a nucleic acid is provided wherein said viral protein is an E2 protein, for example wherein said first nucleic acid fragment comprises a nucleic acid encoding E2 protein from about amino acids 690 to 865 or 692 to 877 or 690 to 1008 or related positions in said pestivirus polyprotein sequence. For simplicities sake the numbering of the C strain sequence is used here for all pestivirus sequences. In fact in other pestivirus sequences the numbering of the $E^{RNS}$ and the E2 proteins in the polyprotein may differ slightly due to length differences in the polyprotein sequences of pestivirus strains. Based on homology, the N and C termini of the E2 or $E^{RNS}$ sequence of any pestivirus strain can, however, easily be determined.

The invention further provides a nucleic acid wherein said protein from another genotype pestivirus is an E2 protein, for example wherein said second fragment comprises a nucleic acid fragment encoding a part of the E2 protein of another pestivirus running from about amino acids 690 to 865 concept" are described. Now that this "exchange concept" approach has been described for generating a CSFV C strain marker vaccine, similar approaches can of course now be followed for any other pestivirus vaccine strain, irrespective of how this strain was generated before it was turned into a marker vaccine strain.

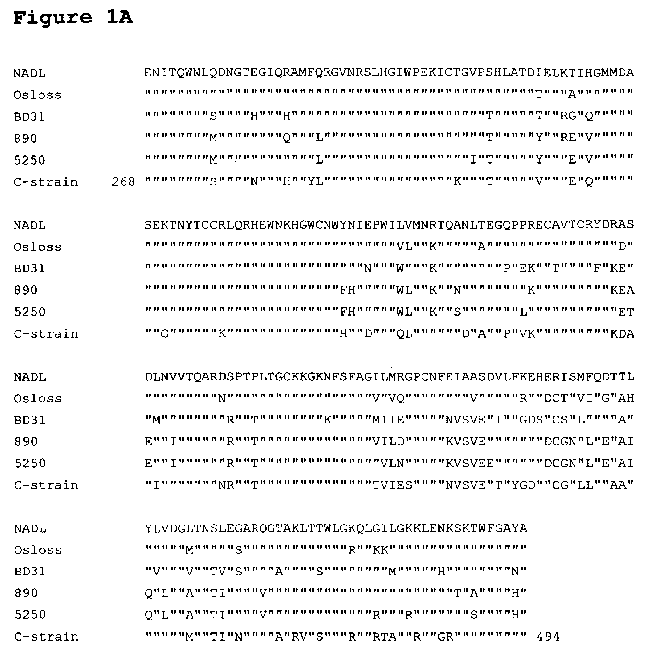

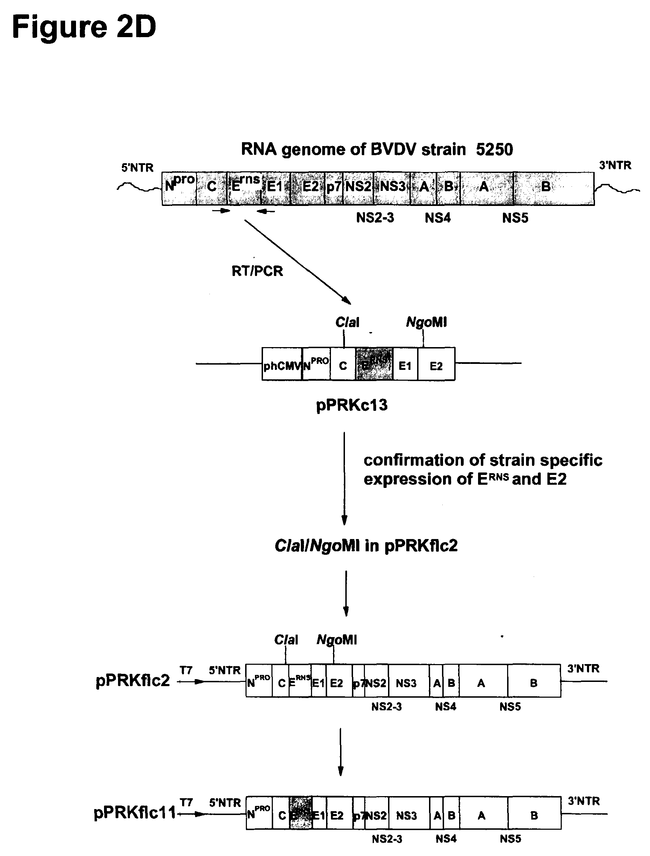

As far as the $E^{RNS}$ protein is concerned a first nucleic acid fragment encoding the part of the polyprotein sequence of the C strain running from amino acid 268 to 494, was replaced by a second nucleic acid fragment encoding the $E^{RNS}$ protein, amino acid 268 to 494 in the polyprotein sequence, of BVDV type II strain 5250 (Fig. 2D). The resulting chimeric C strain virus was designated Flc11. Since the complete sequence of strain 5250 was not available the N and C terminus of the $E^{RNS}$ protein of this strain was determined on the basis of homology between pestivirus sequences (Fig.1A).

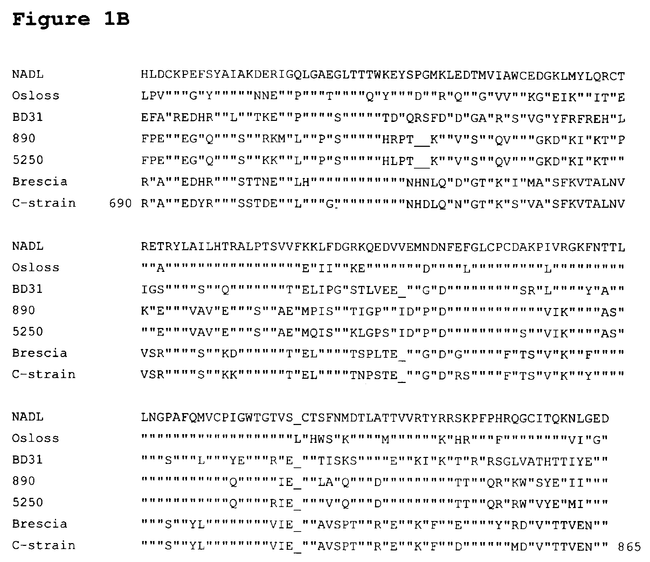

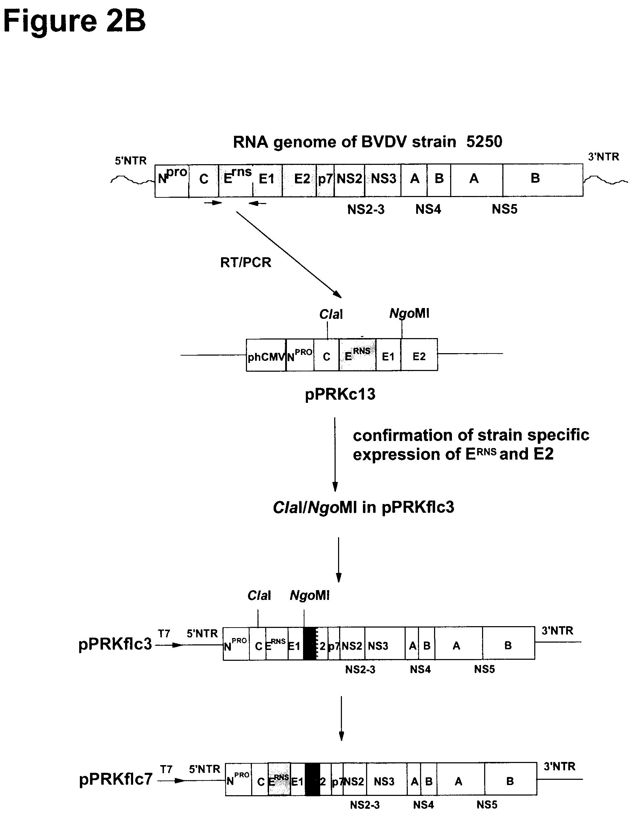

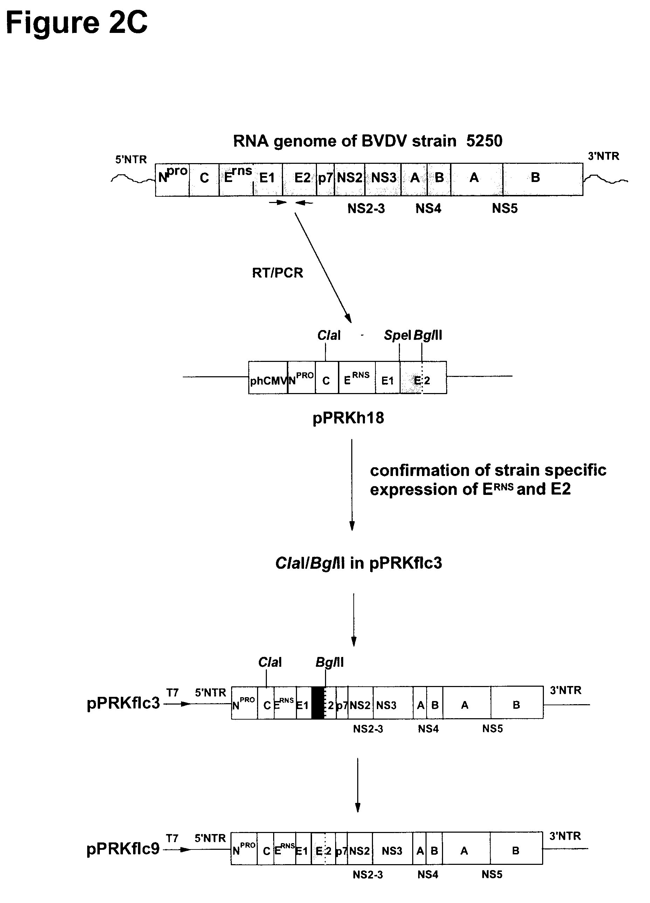

It should be stressed that the antigenic part of $E^{RNS}$, essential to this approach, may be N or C terminally or both, shorter or longer than the sequence exchanged in Flc11. Once established , these parts are as suitable for exchange as the complete $E^{RNS}$ protein, and therefor are also provided. An identical replacement of the $E^{RNS}$ protein of the C strain by the $E^{RNS}$ protein of strain 5250 as described for Flc11 was performed in strain Flc7 (compare fig. 2B). In Flc7 a second first nucleic acid fragment encoding the antigenic N-terminal part of the E2 protein of the C strain running from amino acid 692 to 877 in the polyprotein sequence of the C strain, was replaced by a second nucleic acid fragment encoding the antigenic N-terminal part of the E2 protein of CSFV strain Brescia running from amino acid 692 to 877 in the polyprotein sequence of this strain. As is indicated in figure 1B these fragments essentially comprise an antigenic N-terminal part of the E2 protein of pestiviruses running from amino acid 690 to 865 (van Rijn et al., 1997. Virology 237:337-348). In chimeric virus Flc9 a first nucleic acid fragment encoding this part of the E2 protein of the C strain was replaced by a second nucleic acid fragment encoding the identical part (amino acids 690 to 865, compare fig.1B) of E2 of BVDV type II strain 5250 (Fig. 2C). Another chimeric virus suitable as CSF live virus marker vaccine is virus strain Flc6 in which a first nucleic acid fragment encoding the nearly complete E2 protein running from amino acid 690 to 1008 in the polyprotein sequence of the C strain was replaced by a second nucleic acid fragment encoding E2 of BVDV strain NADL running from amino acid 693 to 1012 in the polyprotein sequence of this strain (Collett et al. 1988. Virology 165:191-199). In conclusion as far as the exchange of E2 protein encoding nucleic acid fragments between pestivirus genotypes for development of pestiviral marker vaccines is concerned, two preferred embodiments have been described in this invention:

1) exchange of the region essentially comprising sequences encoding the antigenic N-terminal half of the protein i.e. amino acid 690 to 877 (numbering of C strain sequence). As shown in this invention, a number of amino acids extra or less at the N (690) and/or C (877) terminus still results in suitable pestivirus marker vaccines.

2) exchange of the region encoding a (nearly) complete E2 protein sequence i.e. amino acid 690 to 1008 (numbering of C strain sequence).

The invention provides a chimeric pestivirus wherein nucleic acid encoding at least a part of at least one pestiviral protein has been replaced by nucleic acid encoding a structurally related part of at least one other pestiviral protein allowing serological discrimination of an animal vaccinated with said vaccine from an animal infected with wild-type pestivirus. A vaccine as provided by the invention preferably comprises a live chimeric pestivirus (and thus preferably is a live vaccine). For a (killed) vaccine comprising a killed chimeric pestivirus substantially more antigenic mass is needed to provide the desired effects, although in principle the effects, relating to protection as well as to serological discrimination, once having been achieved by a killed vaccine, have similar advantages as with a live vaccine.

In a much preferred embodiment, the invention provides a vaccine according to the invention capable of providing sufficient herd immunity to a vaccinated population to reduce the reproduction ratio ($R_0$) of said wild-type pestivirus to at least below 1, thereby allowing sufficient reducton of viral transmission in vaccinated populations to eradicate or control the infection in the population.

In a preferred embodiment nucleic acid or a chimeric pestivirus according to the invention is derived from a pestivirus vaccine strain, such as C strain virus the Japanese GPE- strain virus (Sasahara et al. 1969. Nat. Inst. Of Animal Health Quarterley 9:83-91) or the French Thiverval strain (Aynaud. 1976. EEC Report, EUR 5486, C.E.C. Luxembourg, p.93-96) derived from a classical swine fever virus. Although the invention has been described for a live CSF vaccine strain attenuated by classical techniques, it can equally well be applied to other pestiviral vaccine strains that have been attenuated or generated by e.g. recombinant DNA techniques.

The invention provides a recombinant nucleic acid wherein surprisingly at least a part of a pestiviral protein introduced to allow broad serological discrimination is stably maintained, thus providing the so desired marker characteristics. For example, our experimental results are that Flc6, Flc7, Flc9 and Flc11 stably maintain the heterologous E2 and/or $E^{RNS}$ genes in their otherwise CSFV C-strain genetic make-up. This despite the fact that Flc6 and Flc9 needed adaptation to SK6.T7 cells and grow to lower titres than Flc7, Flc11 and Flc2.

In a preferred embodiment of the invention said chimeric pestivirus comprises Flc7, deposited as number I - 2059, at 31 July 1998, at the CNCM, Paris, France. Strain Flc7 is especially suitable for use as a CSF marker vaccine for emergency vaccination to control outbreaks of CSF in pig areas where there is normally no vaccination against CSF applied and where rapid immunity is required. However, Flc7 is equally suitable as vaccine for prophylactic vaccination in areas where CSF is endemic. Optimal benefit of Flc7 is achieved if the vaccine is used in combination with the serological test which specifically detects antibodies against $E^{RNS}$ of CSFV, and which discriminates the animals infected with the wild type or field CSF virus from the uninfected animal or the animal vaccinated with Flc7. In this way Flc7 allows the specific detection of the CSFV infected animal or herd in the vaccinated population, which animal or herd can than be specifically removed.

In a preferred embodiment of the invention said chimeric pestivirus comprises Flc9, deposited as number I - 2060, at 31 July 1998, at the CNCM, Paris, France.

Strain Flc9 is especially suitable for use as a CSF marker vaccine for emergency vaccination to control outbreaks of CSF in pig areas where there is normally no vaccination against CSF applied and where rapid immunity is required. However, Flc9 is equally suitable as vaccine for prophylactic vaccination in areas where CSF is endemic. Optimal benefit of Flc9 is achieved if the vaccine is used in combination with the serological test which specifically detects antibodies against E2 of CSFV, and which discriminates the animals infected with the wild type or field CSF virus from the uninfected animal or the animal vaccinated with Flc9. In this way Flc9 allows the specific detection of the CSFV infected animal or herd in the vaccinated population, which animal or herd can than be specifically removed.

In a preferred embodiment of the invention said chimeric pestivirus comprises Flc11, deposited as number I - 2061, at 31 July 1998, at the CNCM, Paris, France. Strain Flc11 is especially suitable for use as a CSF marker vaccine for emergency vaccination to control outbreaks of CSF in pig areas where there is normally no vaccination against CSF applied and where rapid immunity is required. However, Flc11 is equally suitable as vaccine for prophylactic vaccination in areas where CSF is endemic. Optimal benefit of Flc11 is achieved if the vaccine is used in combination with the serological test which specifically detects antibodies against $E^{RNS}$ of CSFV, and which discriminates the animals infected with the wild type or field CSF virus from the uninfected animal or the animal vaccinated with Flc11. In this way Flc11 allows the specific detection of the CSFV infected animal or herd in-the vaccinated population, which animal or herd can than be specifically removed.

In a preferred embodiment of the invention said chimeric pestivirus comprises Flc6, deposited as number I - 2058, at 31 July 1998, at the CNCM, Paris, France. Strain Flc6 is especially suitable for use as a CSF marker vaccine for emergency vaccination to control outbreaks of CSF in medium pig areas where there is normally no vaccination against CSF applied and where rapid immunity is required. However, Flc6 is equally suitable as vaccine for prophylactic vaccination in areas where CSF is endemic. Optimal benefit of Flc6 is achieved it the vaccine is used in combination with the serological test which specifically detects antibodies against E2 of CSFV, and which discriminates the animals infected with the wild type or field CSF virus from the uninfected animal or the animal vaccinated with Flc6. In this way Flc6 allows the specific detection of the CSFV infected animal or herd in the vaccinated population, which animal or herd can than be specifically removed.

The invention furthermore provides a method for controlling and/or eradicating a wild-type pestivirus infection comprising vaccinating at least a part of an animal population with a vaccine according to the invention and further comprising testing at least a part of said population for the presence of antibodies specific for said wild-type virus.

Animals infected with an field virus develop antibodies against an immunodominant part of that virus. An ELISA or neutralisation test (differentiating tests) as provided in the experimental part of this description which detects these antibodies is used for the detection of infected animals, which subsequently are removed from a population. The use of a pestivirus marker vaccine as provided by the invention wherein said immunodominant part has been modified or replaced allows serological discrimination between vaccinated and field-virus infected animals, and thereby a controlled elimination of the virus. These differentiating tests are also used for monitoring the status of wild-type pestivirus infections and transmission in a population during or after vaccination campaigns. Using a vaccine as provided by the invention capable of providing sufficient herd immunity to a vaccinated population to reduce said wild-type virus transmission to a reproduction ratio of < 1 greatly facilitates control and/or eradication. Eventually, the aim is to reach a field or wild-type virus free status of a herd. Vaccination can then be discontinued and a serological surveillance program to guard this status should come into force.

When an outbreak of CSFV is notified, eradication of the virus usually occurs by a stamping out policy. The availability of a CSFV marker vaccine now allows vaccination with such a vaccine as an additional tool to control the CSFV outbreak. A possible way to proceed is that all pigs of the infected herd will be destroyed and all pigs on farms in a certain zone around the infected herd are vaccinated with a marker vaccine as provided by the invention. The vaccine preferably should be administered within a short time (e.g. 2 weeks). The vaccination can possibly be followed by a follow-up vaccination, three to four weeks after the first vaccination.

After vaccination, a surveillance period will be started. During this period, which might take at least 2 months, all herds in the zone will be screened regularly for clinical symptoms of CSF, and serum samples will be collected. These serum samples will be tested in the differentiating tests for antibodies directed against wild-type CSFV. Herds with pigs with antibodies directed against wild-type CSFV will be destroyed. Herds with pigs without antibodies directed against wild-type CSFV during a prescribed period should be declared negative for CSFV. The zone should be declared free of CSFV when during a prescribed period no antibodies against wild-type CSFV are detected on all herds in the surveillance zone. After the lift of movement restrictions, the CSFV-negative but vaccinated pigs can be traded and slaughtered. Policy makers, however, will further decide about the implementation of the vaccine and about the measures taken after vaccination, for example depending on regional or political preferences.

Likewise control and/or eradication measures are provided for BDV and BVDV. The invention is further explained in the experimental part of the description without limiting the invention.

**Experimental part**

**Example 1**

**Construction and characterisation of chimeric pestiviruses**

**Cells and virus**

Swine kidney cells (SK6-M, EP 0 351 901 B1) were grown in Eagle's basal medium containing 5% fetal bovine serum, glutamine (0.3 mg/ml), and the antibiotics penicillin (200 U/ml), streptomycin (0.2 mg/ml), and mycostatin (100 U/ml). Fetal bovine serum was tested for the absence of BVDV and BVDV antibodies as described previously (Moormann *et al*. 1990. Virology **177**:184-198). SK-6 cells expressing bacteriophage T7 RNA polymerase (SK6.T7; H.G.P.van Gennip *et al*. 1998. submitted) were grown in the same medium supplemented with 12.5 times diluted histidinol stock (125 mM histidinol in 100 mM HEPES).
Recombinant or chimeric C-strain viruses were grown and prepared as described earlier (Moormann *et al*. 1996. J. Virol.**70**:763-770) with slight modifications. The growth medium of SK6 cells was changed in supplemented Eagle's basal medium. Virus stocks were prepared by passaging the virus three times on SK6 cells. The virus stocks had titres of about $10^{4,0}$-$10^{6,5}$ $TCID_{50}$/ml. BVDV strain 5250 of porcine origin (Hooft van Iddekinge *et al*., 1992. Vet. Microbiol. **30**:21-34; Wensvoort *et al*., 1989. Vet. Microbiol. **20**, 291-306) was biologically cloned three times by end point dilution on bovine turbinate (BT) cells and was also grown on SK6 cells as described above.

**Isolation of RNA from infected SK6 and BT cells**

Intracellular RNA from cells infected with the recombinant C-strain viruses was isolated essentially as described (Moormann *et al*. 1990. Virology **177**:184-198).
Briefly, monolayers of SK6-M cells were infected with virus at a multiplicity of infection (m.o.i.) of 0.1. Cells were incubated for 1.5 h at 37°C, and fresh medium was added.
After 4 days cells were washed twice with ice cold phosphate buffered saline (PBS), and lysed in ice-cold lysisbuffer (50 mM Tris-HCl pH 8.2, 0.14 M NaCl, 2 mM $MgCl_2$, 5 mM DTT, 0.5%[v/v] NP-40, 0.5%[w/v] Na-deoxycholate, and 10 mM vanadyl ribonucleoside complexes (New England Biolabs)). The lysates were centrifuged (4 °C, 5 min, 4000x $g$) and the supernatant was treated with proteinase K (250 μg/ml, final concentration) for 30 min at 37 °C, extracted twice with phenol, chloroform, and isoamylalcohol (49:49:2), and extracted once with chloroform and isoamylalcohol (24:1). RNA was stored in ethanol.
RNA from strain 5250 from BT cells was isolated as described previously (Hooft van Iddekinge *et al*., 1992. Vet. Microbiol. **30**:21-34).

**Cloning and sequencing of cDNA of the genes encoding E$^{RNS}$ and E2 of BVDV strain 5250**

The E2 gene of BVDV strain NADL was cloned and sequenced as described (van Rijn *et al.* 1997. Virology **237**:337-348), resulting in plasmid pPRKbvd10.
The antigenic region of the E2 gene of strain 5250 was cloned and sequenced as described (van Rijn *et al*. 1997. Virology **237**:337-348), resulting in plasmid pPRKbvd77.
RNA from BT cells infected with BVDV strain 5250 was isolated as described above and a fragment covering the E$^{RNS}$ gene was amplified from cDNA by reverse transcription-PCR (RT-PCR) essentially as described (van Rijn *et al*. 1994. J. Virol. **68**:3934-3942) using forward primer p343 (5'-CCCGGGATCCAAAAAGCCCTGTTGGC[A,T]TGGGC-3') and reverse primer p305 (5'-GGGGTGCAGTTGTTTGTATCCA-3'). The blunt PCR-products were cloned in pGEM4z-blue. The nucleotide sequence of E$^{RNS}$ of strain 5250 was determined by thermocycle sequencing (Perkin Elmer/Applied Biosystems Division) on two independently amplified and cloned fragments. Deduced amino acid sequences were compared with those of published BVDV I and II and BDV strains with the MacMolly-tetra multialignment program.

**Construction of hybrid full-length DNA clones pPRKflc6, pPRKflc7, pPRKflc9 and pPRKflc11**

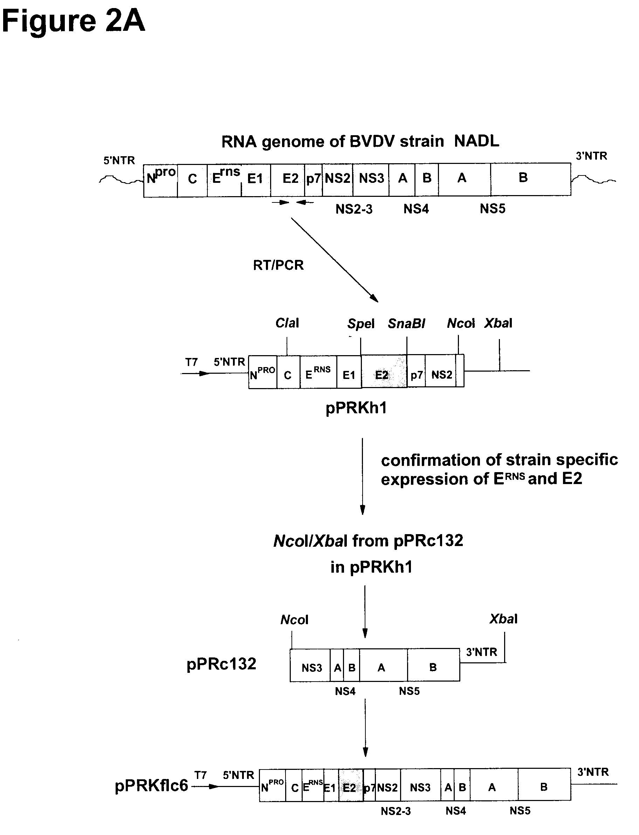

pPRKflc6 is a full-length clone containing the C-strain genome in which the E2 gene between the *Spe*I and *Afl*II sites of pPRKflc2 (previously named pPRKflc-133, Moormann *et al*., 1996. J. Virol. **70**:763-770), was replaced with the same region of BVDV strain NADL (Fig. 2A). The *Spe*I-*Sna*BI fragment of pPRKbvd10 (van Rijn *et al*. 1997. Virology **237**:337-348) was cloned in pPRc144, which is a derivative of pPRc129 (WO 95/35380) in which the *Spe*I site in the vector was mutated to make this site unique. The resulting plasmid, designated pPRKh1, was transfected into SK6.T7 cells and tested for expression of C-strain E$^{RNS}$ and NADL E2 as described previously (van Rijn *et al*. 1993. J.

Gen. Virol. **74**:2053-2060). C-strain E$^{RNS}$ expression was detected with Mab C5 (Wensvoort, G. 1989. Epitopes on structural proteins of hog cholera (swine fever) virus. PhD thesis, State University of Utrecht), and NADL E2 expression was detected with Mab CT6 (Bolin *et al*. 1988. Arch. Virol. **99**, 117-123). Finally, the *Nco*I-*Xba*I fragment of pPRc132 (WO 95/35380) was inserted in *Nco*I-*Xba*I digested pPRKh1, resulting in the hybrid full-length plasmid pPRKflc6 (Fig 2A).

pPRKflc7 is a derivative of pPRKflc3 (previously named pPRKflc-h6, Moormann *et al*., 1996. J. Virol. **70**:763-770) in which the E$^{RNS}$ gene of CSFV strain C between amino acids 267-495 (numbering of CSFV strain C; EMBL nr. Z46258) was replaced with the same region of BVDV strain 5250 (Fig. 2B). To this end, the E$^{RNS}$ gene was PCR amplified. Plasmid pPRKbvd37 (unpublished), containing the E$^{RNS}$ gene from BDVD strain 5250, was used as template DNA and amplified with forward primer p936 (5'-CCGAGAACATCACCCAGTGG-3') and reverse primer p937 (5'-CATGTGCTCCAAACCATGAT-3'). The PCR fragments were phosphorylated with T4 polynucleotide kinase (New England Biolabs) and isolated from an agarose gel. The isolated fragments were cloned in a calf intestinal phosphatase (New England Biolabs)-treated *Stu*I site of expression vector pPRKc5 (Hulst *et al*. 1998. J. Virol. **72**:151-157). Clones in which the 5250 E$^{RNS}$ gene were inserted in the correct orientation were transfected into SK6 cells and tested for expression of 5250 E$^{RNS}$ and C strain E2 as described above. E$^{RNS}$ expression was detected with Mab WB433 (Paton *et al*. 1994. Arch. of Virol. **135**:241-252), and C-strain E2 expression was detected with Mab b3 (Wensvoort, G. 1989. J. Gen. Virol. **70**:2865-2876). From clone pPRKc13, which expressed both 5250 E$^{RNS}$ and C-strain E2, a *Cla*I-*Ngo*MI fragment was inserted in the *Cla*I-*Ngo*MI digested pPRKflc3, resulting in full-length hybrid clone pPRKflc7 (Fig. 2B).

pPRKflc9 is a full-length clone containing the C-strain genome in which the antigenic region of E2 between the *Spe*I and *Bgl*II sites of pPRKflc3, was replaced with the same region of BVDV strain 5250 (Fig. 2C). To this end, the *Spe*I-*Sal*I fragment of pPRKbvd77 (van Rijn *et al*. 1997. Virology **237**:337-348) was cloned in expression vector pPRc83. This vector, a pEVhisd12 derivative (Peeters *et al*. 1992. J. Virol. **66**:894-905), contains the structural genes of CSFV strain C (N$^{pro}$-C-E$^{RNS}$-E1-E2, amino acids 5 to 1063 of the sequence of CSFV strain C) (Moormann *et al*., 1996. J. Virol.**70**:763-770). The resulting clone, designated pPRKh18, was transfected into SK6 cells and tested for expression of C-strain E$^{RNS}$ and 5250 E2 as described above. Expression of C-strain E$^{RNS}$ was detected with Mab C5 (see above), and expression of 5250-E2 was detected with Mab WB166 (Paton *et al*. 1992. Virology **190**:763:772). The *Cla*I-*Bgl*II fragment of pPRKh18 was inserted in *Cla*I-*Bgl*II digested pPRKflc3, resulting in the hybrid full-length plasmid pPRKflc9 (Fig. 2C).

pPRKflc11 is a full-length clone containing the C-strain genome in which the E$^{RNS}$ gene of CSFV strain C between amino acids 267-495 (numbering of CSFV strain C; EMBL database nr Z46258) was replaced with the same region of BVDV strain 5250 (Fig. 2D). From clone pPRKc13, also mentioned above (Fig. 2B), a *Cla*I-*Ngo*MI fragment was inserted in the *Cla*I-*Ngo*MI digested plasmid pPRKflc2, resulting in the hybrid full-length clone pPRKflc11 (Fig. 2D).

**Isolation of recombinant viruses Flc6, Flc7, Flc9 and Flc11**

Plasmid DNA from pPRKflc6, pPRKflc7, pPRKflc9 and pPRKflc11 was purified on columns (Qiagen) and linearized with *Xba*I. The DNA was extracted with phenol-choroform, precipitated with ethanol, and dissolved in water. 25 ml of Optimem-I containing 1 mg Lipofectine (Gibco-BRL) was preincubated for 45 min at room temperature. Linearized DNA (200 ng) was diluted in 25 ml Optimem-I (Gibco-BRL) and mixed with the preincubated lipofectine mixture and incubated for 15 min at room temperature. SK6.T7 cells, grown in 2 cm$^2$ tissue culture plates, were washed once with Optimem-I. Fresh Optimem-I was added (0.2 ml), followed by the DNA transfection mixture. After 5 h of incubation at 37°C, the transfection mixture was removed and the wells were supplied with fresh medium. The cells were incubated for 4 days at 37°C, after which the transfection supernatant was stored at -70°C. Cells were immunostained with Mabs C5 (for Flc6 and Flc9) and b3 (for Flc7 and Flc11). The medium collected from wells in which expression was observed was applied onto fresh SK6 or SK6.T7 cells to determine the presence of infectious virus. After four days, the monolayers were fixed and immunostained as described above.

**Growth kinetics of Flc9 and Flc11**

Growth kinetics of the viruses Flc9 and Flc11 were determined in SK6 cells. Subconfluent monolayers in T25 flasks (Costar) were infected at a multiplicity of infection of 0.1. Viruses were adsorbed for 1.5 h. Before cells were supplied with fresh medium, the first sample at time point zero was collected. Virus titres were determined separately from supernatants and cell lysates. To determine the virus titre in cells, cells were freeze/thawed twice at -70°C in 2 ml of growth medium and clarified for 10 min at 5000 x *g* at 4°C. Virus titres (log TCID$_{50}$ per millilitre) were determined at 0, 6, 24, 48, 72, 96, 122 and 144 h after infection.

**Characterization of Flc6, Flc7, Flc9 and Flc11**

The E$^{RNS}$ genes of Flc7 and Flc11, and the E2 genes of Flc6 and Flc9 were sequenced. Cytoplasmic RNA of SK6 cells infected with the respective viruses was isolated as described above. DNA fragments covering the E$^{RNS}$ genes of Flc7 and Flc11 and the E2 genes of Flc6 and Flc9 were amplified by RT-PCR and analysed on a 1.5% agarose gel in 1xTAE, and purified on Costar Spin-X columns. Sequences of the purified PCR fragments were determined by PCR cycle sequencing using the dRhodamine dye terminator ready reaction cycle sequencing kit (PE/ABD) according to the manufacturers conditions with flanking and internal primers and analysed on a 310 ABI PRISM genetic analyser. In addition, viruses were characterised by an immunoperoxidase monolayer assay (Wensvoort, G. 1986. Vet. Microbiol. **12**, 101-108). For this test, SK6 cells were infected with viruses Flc2, Flc6, Flc7, Flc9, Flc11 and 5250. After incubation for 4 days at 37°C, monolayers were immunostained with Mabs specific for CSFV E2 (Mabs b6 and c2), BVDV E2 (Mabs WE166 and CT6), C-strain E$^{RNS}$ (Mab C5), or BVDV E$^{RNS}$ (Mab WB433).

**Results**

**Cloning and sequencing of cDNA of the genes encoding E$^{RNS}$ and E2 of BVDV strain 5250**

The cloning and sequencing of the N-terminal part of E2 of BVDV strain 5250 has been described by Van Rijn *et al*., 1997. (Virology 237:337-348). The cloning and sequencing of the E$^{RNS}$ gene of this strain has not been published and is here described for the first time. Established and published amino acid sequences of 5250 E2 and E$^{RNS}$ were aligned with those of BVDV type I strains NADL (Collett *et al*. 1988. Virology **165**:191-199) and Osloss (Renard *et al*. 1987. European patent application 86870095.6), BVDV type II strain 890 (Ridpath and Bolin. 1995. Virology **212**:39-46), CSFV strain C (Moormann *et al*. 1996. J. Virol. **70**:763-770), CSFV strain Brescia (Moormann *et al*. 1990. Virology **177**:184-198) and BDV strain BD31 (Sullivan *et al*., 1997. Virus Res. **47**:19-29). This alignment clearly demonstrated that the 5250 E$^{RNS}$ and E2 sequences are most homologous with those of BVDV strain 890 (Figs. 1A and 1B), and confirm the data of van Rijn *et al*. 1997 (ibid), who on basis of sequence comparison of E2, demonstrated that 5250 belongs to the BVDV type II category of pestiviruses.

**Construction and isolation of Flc6, Flc7, Flc9 and Flc11; production and growth kinetics of Flc9 and Flc11.**

The construction of plasmids pPRKflc6, pPRKflc7, pPRKflc9 and pPRKflc11 which contain full-length DNA copies of the RNA genomes of CSFV C-strain viruses in which the E$^{RNS}$ or E2 genes or both have been substituted, is shown in figures 2A-D. For recovery of recombinant viruses, full-length cDNA plasmids were transfected into SK6.T7 cells endogenously expressing the bacteriophage T7 RNA polymerase gene.
Infectious virus could be recovered from supernatants of SK6.T7 cells transfected with pPRKflc7 and pPRKflc11 but not from cells transfected with pPRKflc6 and pPRKflc9. However, because SK6.T7 cells transfected with pPRKflc6 and pPRKflc9 were positive after immunostaining, cells transfected in parallel were trypsinized, diluted 20-fold in Eagle's supplemented medium and grown for four days in two T25 flasks. The cells were passaged several times in the same way. After each passage, one T25 flask was freeze/thawed twice and the cell suspensions were clarified at 5000 x *g* for 10 minutes at 4°C. The virus titres of the clarified supernatants were determined by end point dilution. After three passages on SK6.T7 cells, virus titres increased to a maximum of $10^4$ TCID$_{50}$/ml for Flc 6 and Flc9. In the same way virus stocks of about $10^5$ TCID$_{50}$/ml of Flc7 and Flc11 were prepared.
Viruses Flc9 and Flc11 were further propagated on SK6 cells as described in the section "Cells and Virus". Virus stocks of Flc9 reached titres of $10^5$-$10^6$ TCID$_{50}$/ml, whereas virus stocks of Flc11 reached titres of $10^6$-$10^7$ TCID$_{50}$/ml.

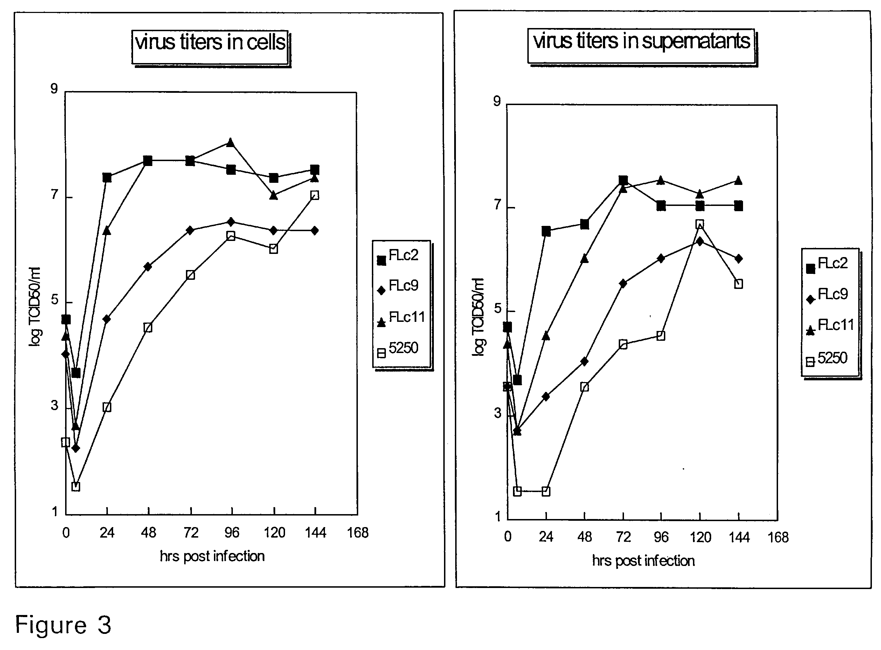

Since Flc9 needed adaptation to SK6.T7 cells and seemed to grow to log10 lower virus stock titres, growth kinetics of Flc9, Flc11 and the parent viruses 5250 and Flc2 were compared. As is shown in figure 3 the multistep growth curves of Flc9 and 5250 are very similar, and indicate a slower growth rate and about log10 lower virus titre plateau levels for these viruses, compared to Flc2 and Flc11. Also the growth curves of Flc2 and Flc11 are very similar, as are their virus titre plateau levels. This suggests that the growth rate of CSFV based chimeric pestiviruses in SK6 cells is more dependent on the presence in these constructs of CSFV E2 than on the presence of CSFV E$^{RNS}$. Probably this effect is exerted at the level of infection.

**Characterization of Flc6, Flc7, Flc9 and Flc11.**

Characterization of Flc6, Flc7, Flc9 and Flc11 at the molecular level was performed by sequencing their E2 (Flc6, Flc9) or E$^{RNS}$ (Flc7, Flc11) genes. No sequence differences resulting in amino acid changes between the E2 gene of Flc9 and the E$^{RNS}$ genes of Flc7 and Flc11, and the sequences of E2 and E$^{RNS}$ of 5250, from which they were derived, were observed. The same result was found when the sequence of the E2 gene of Flc6 was compared with published NADL E2 sequences (Collett *et al*. 1988. Virology **165**:191-199; van Rijn *et al*. 1997. Virology **237**:337-348). Furthermore, Flc6, Flc7, Flc9 and Flc11 were characterized by their specific reaction with a panel of CSFV and BVDV specific Mabs directed against E$^{RNS}$ and E2 (Table 1). Flc9 and strain 5250, containing the E2 gene of BVDV type II strain 5250, specifically react with Wb166, a BVDV E2 specific Mab. Strains Flc9 and 5250 can be discriminated from NADL and Flc6, containing the E2 gene of BVDV type I strain NADL, by the specific reaction of another BVDV E2 specific Mab, CT6, with the latter two strains. As expected, strains Flc6, Flc7, Flc9, NADL, 5250, and Brescia do not react with Mab C2 which specifically reacts with strains Flc2 and Flc11, both containing the E2 gene of CSFV strain C. Mab b6, which recognizes an epitope on E2 of CSFV strain Brescia specifically reacts with strains Flc7 and Brescia. Further analysis with pestivirus strain specific E$^{RNS}$ Mabs resulted in a more refined antigenic typing of each of the above described (recombinant) pestivirus strains. The BVDV E$^{RNS}$ specific Mab WB433 specifically reacts with strains Flc7, Flc11 and 5250, since these strains contain the BVDV specific E$^{RNS}$ gene of strain 5250. In contrast, these strains do not react with the CSFV specific E$^{RNS}$ Mab C5, which specifically reacts with strains Flc2, Flc6 and Flc9, containing the E$^{RNS}$ gene of CSFV strain C.

Combining the data of the Mab typing on E$^{RNS}$ and E2 resulted in unique reaction profiles with the Mabs for each of the strains in Table 1.

In conclusion, these findings show that Flc6, Flc7, Flc9 and Flc11 stably maintain the heterologous E2 and/or E$^{RNS}$ genes in their otherwise CSFV C-strain genetic make-up. This despite the fact that Flc6 and Flc9 needed adaptation to SK6.T7 cells and grow to lower titres than Flc7, Flc11 and Flc2.

**Example 2 (animal experiment: 298-47042-00.22/97-11) Pigs vaccinated with Flc9 and Flc11 are protected against a lethal challenge with virulent CSFV strain Brescia, and can be discriminated from infected animals by CSFV-specific E2 or E$^{RNS}$ serology.**

### Materials and Methods

### Animals

Specified-pathogen-free (SPF) pigs of 6-7 weeks of age were obtained from the SPF herd of the ID-DLO. Pigs were randomly divided in groups, and housed in separate stables of the high containment facilities of ID-DLO. The animals were left to acclimatise for four days before the first treatment. The animals were fed once a day, in a trough, with complete food pellets, and could drink water from a nipple ad libitum.

### Vaccination and challenge

On arrival, 27 SPF pigs were randomly divided in three groups: two groups of 10 pigs (A, B), and one group of 7 pigs (C). The pigs in group A were vaccinated with strain Flc 9; the pigs in group B were vaccinated with strain Flc 11. The vaccines were dissolved in an oil-in-water emulsion (DOE) (Hulst *et al*., 1993. J.Virol. **67**:5435-5442). Each animal received 2 ml of the vaccine intramuscularly in the neck behind the ear. The pigs in group C remained unvaccinated.

Group A: Flc 9 (10)
Group B: Flc 11 (10)
Group C: controls (7)

Four weeks after vaccination, five animals in the groups A and B, and three animals in groups C were separated from their group and placed in a different, separate part of their stable. These pigs were challenged intranasally with 100 50% lethal doses (= 100 LD$_{50}$) of CSFV strain Brescia 456610 (Terpstra and Wensvoort, 1988. Vet. Microbiol. **16**:123-128). The pigs returned to their original group 24 h later. The pigs remained together during seven weeks.

Viral contents of the challenge inoculum were determined by titration of a sample taken after return from the stable.

### Clinical observations

The pigs were checked daily by the animal technicians, abnormal findings were recorded and if necessary the supervising veterinarian was called. Each group was observed at least 15 minutes per day before and during feeding and cleansing of the stable.

A reduction in food uptake of the group or an individual animal was noted. Body-temperatures were recorded during several days before and up to 20 days after challenge.

**Blood analysis after challenge**

EDTA-blood samples were collected on days 0, 2, 4, 7, 9, and 11 after challenge to monitor changes of leucocyte and trombocyte numbers in the blood. A decrease in the number of leucocytes (leucopenia) and thrombocytes (thrombocytopenia) is one of the typical signs of CSF. Normal cell counts for white blood cells and thrombocytes in conventional swine range between 11-23 $10^9$/l and 320-720 $10^9$ /l, respectively. For SPF pigs these values are a bit lower: 5-12 $10^9$/l and 200-700 $10^9$/l, respectively. Both ranges mentioned vary in each pig. The bloodcell analyses were performed with a Medonic$^®$ CA 570 coulter counter. Leucopenia and thrombocytopenia were defined as cell/platelets counts considerably lower than the minimum number mentioned above, preferably for more than one day.

**Virus isolation and viral antigen detection**

Virus isolation: Peripheral blood leukocytes were extracted from EDTA-blood samples taken on day 0, 2, 4, 7, 9, and 11 after challenge to monitor viraemia. The samples were stored at -70 °C. The presence of CSFV in the leucocytes was examined as follows. In a M24 plate (Greiner), 300 μl (containing approximately $5x10^6$ cells) of a swine kidney cell (SK6) suspension was added to each well and cultured at 37 °C and 5 % $CO_2$ in a humid chamber for 24 h. After 24 h, the medium was removed and 300 μl of an undiluted freeze/thawed leucocyte sample was added per well. After one hour of incubation at 37° C and 5% CO2, the sample was removed. The monolayer was then washed by adding and removing 400 μl of culture medium (Eagle basal medium). Subsequently, 800 μl of culture medium (Eagle basal medium with 5 % fetal bovine serum (FBS), free of pestivirus antibodies, and 1 % of an antibiotic stock ( containing ; glutamine (0.3 mg ml$^{-1}$), penicillin (200 units ml$^{-1}$), streptomycin (0.2 mg ml$^{-1}$) and mycostatin (100 U ml$^{-1}$.)) was added per well. After four days, the monolayers were washed in 10% NaCl solution, dried for 1 h at 80° C, incubated with a buffered solution containing CSFV specific conjugated antibodies, washed and stained. The monolayers were read microscopically for stained cells. Results were expressed as positive or negative for virus.

IFT: At post-mortem, tissue samples were collected from tonsil, spleen, kidney, and ileum, and were tested by direct immunofluorescent technique (Ressang and De Boer, 1967. Tijdschrift voor Diergeneeskunde **92**:567-586) for the presence of viral antigen. Cryostat sections (4 μm thick, two per organ) from these tissue samples were fixed and incubated with a polyclonal swine anti-pestivirus FITC-conjugated serum. After washing, the sections were read under a fluorescence microscope. Results were expressed as positive (= fluorescence) or negative (= no fluorescence).

**Serological response**

Serum blood samples of all pigs except the controls were collected at 0, 2, and 3 weeks after vaccination and at death (5 weeks after challenge). Samples were stored at -20 °C and assayed in a CSFV specific (Terpstra and Wensvoort. 1984. Vet. Microbiol. **33**:113-120) and 5250 specific (de Smit, unpublished) virus neutralization test (NPLA), in the Ceditest$^®$ ELISA for detecting CSFV specific antibodies against E2 (Colijn et al., 1997. Vet. Microbiol. **59**:15-25), and in a Ceditest$^®$ ELISA for the detection of antibodies against E$^{RNS}$ (de Smit et al., in prep).
CSFV specific neutralizing antibody titres in serum were determined in a microtitre system. Serial twofold dilutions of serum were mixed with an equal volume of a CSFV (strain Brescia) suspension which contained 30-300 $TCID_{50}$. After incubation for 1 hour at 37 °C in a $CO_2$ - incubator approximately 25,000 PK-15 cells per well were added. After four days, the microtitre plates were treated as mentioned above and read microscopically. The CSFV neutralizing titre was expressed as the reciprocal of the highest dilution that neutralized all virus.
Neutralizing antibody titres against 5250 in serum were determined similarly using the BVDV strain 5250 as antigen. The CSFV E2-ELISA was performed according to the instructions of the manufacturer (Colijn et al., 1997. ibid). The CSFV E$^{RNS}$-ELISA was performed as follows (de Smit et al, in prep). Test sera (30 μl) are pre-incubated with CSFV E$^{RNS}$-antigen (70 μl of a working dilution of baculovirus expressed E$^{RNS}$ of CSFV strain Brescia) in a 96-wells non-coated microtitre plate containing 45 μl of ELISA buffer for 30 min at 37°C.
Thereafter 50 μl of this pre-incubation mix is added to a microtitre plate coated with the E$^{RNS}$-specific monoclonal antibody 137.5, and containing 50 μl of a working solution of the peroxidase$^®$ conjugated E$^{RNS}$-specific monoclonal antibody 140.1.1. The plates are incubated for 1 h at 37°C, washed six times with 200 μl of washing solution, and incubated for 30 min at room temperature with 100 μl of a ready-to-use chromogen (3,3',5,5'-tetramethylbenzidine)/substrate solution. The colour reaction is stopped by adding 100 μl of a 0.5 M $H_2SO_4$ solution, and the optical density was measured at 450 nm using an Easy Reader$^®$ spectrophotometer (SLT Vienna).

**Transmission of virus**

The reproduction ratio R was used as measure for transmission. R is the number of secondary infections caused by one infectious animal. The seroconversion, body temperature, leucocyte- and thrombocyte counts, viremia, and antigen detection in tissues of the contact pigs were used to detect virus transmission by the vaccinated pigs after challenge. *R* was estimated by determining the number of contact infections and subsequent analysis using a stochastic mathematical model (De Jong and Kimman, 1994. Vaccine **12**: 712-766). *R* is an indication of whether the infection will spread or will fade out. When *R* < 1, the infection will disappear from the population; when *R* > 1, the infection may spread. *R* is estimated as follows:

$$R = \frac{N}{T} \sum_{i=S_T+1}^{S_0} \frac{1}{i}$$

In this formula, $S_0$ is the initial number of contact pigs; N is the total number of pigs; T is the number of pigs that have been infectious in the course of the experiment ($T = x + I_0$); $S_T$ is the number of pigs that are still susceptible at the end of the experiment.

## Results

### Clinical observation, viral antigen detection, leukocyte/trombocyte counts

After vaccination none of the animals developed clinical signs or fever. After challenge, all control pigs (group C) developed fever, and other clinical signs of CSF like anorexia, paralysis, thrombocytopenia and leucopenia (Table 2). The three inoculated pigs (nos. 621, 622, 625) developed fever two days after challenge. The four contact pigs developed fever after 7 days (624) or 8 days (623, 626, 627) after contact with the inoculated pigs. All pigs were killed in a moribund state 13 days after challenge.

In group A (Flc9), all inoculated pigs (nos. 602, 606, 608, 609, 610) developed a mild fever (40 °C < T <41 °C) for one or more days, starting 3 days after challenge. In this group three contact-exposed pigs developed a mild fever, no. 604 at six days, no. 605 at seven days, and no. 607 at three days after contact. Two contact pigs did not develop fever. In group B (Flc11), only one inoculated pig (605) had a very mild fever (40 °C) for one day. None of the other inoculated pigs nor the contact-exposed pigs developed fever.

In the control group (group C), all pigs developed trombocytopenia (< 180 cells/l); only three pigs (nos. 622, 623, 627) developed leucopenia (<4.8x10⁹ cells/l) , but in the other 4 pigs the number of leucocytes decreased (Table 2). None of the vaccinated pigs, either in group A (Flc9) or B (Flc11), developed leucopenia or trombocytopenia.

In the control group (group C), virus was detected in the leucocytes of all pigs, either inoculated or contact-exposed. Moreover, after killing of the pigs, all tissues of all pigs were IFT-positive (Table 2).

In both groups A and B (Flc9 and Flc11, respectively), no virus was detected in the leucocytes. Moreover, the tissues of all pigs were IFT-negative at the end of the experiment.

### Serological response

All pigs in the control group (group C) died of CSF before developing any serological response.

After vaccination of the pigs in group A (Flc9), no CSFV-specific antibodies were detected (Table 3) with the E2-ELISA, whereas all pigs showed a low background inhibition percentage in the E$^{RNS}$ ELISA (Table 4). This finding was consistent with the NPLA results: all vaccinated pigs remained negative for neutralising antibodies against CSFV (Table 5), and some (602, 603, 604, 607 and 608) developed low levels of neutralising antibodies against BVDV strain 5250 (Table 6). The latter was as expected since Flc9 contains the E2 gene, encoding the major antigenic protein of pestiviruses, of strain 5250.

After challenge inoculation, a high inhibition percentage in the E2-ELISA was observed in all inoculated pigs, and in two contact-exposed pigs (nos. 601, 603) (Table 3). Four of the inoculated pigs (nos. 606, 608, 609, 610) and one contact pig (no. 601) showed a high inhibition percentage in the E$^{RNS}$ ELISA (Table 4). The fifth inoculated pig showed a lower, but positive inhibition percentage in the E$^{RNS}$ ELISA, and the four remaining contact pigs showed intermediate inhibition percentages, considered negative.

In the neutralising antibody assays, all inoculated pigs and one contact pig (no. 601) showed high titres against CSFV. Two contact pigs (603, 607) showed an intermediate titre, and two remaining contact pigs did not develop antibodies against CSFV (Table 5). In the NPLA against 5250, four of the inoculated pigs (nos. 606, 608, 609, 610) developed high titres against 5250 after inoculation (Table 6). The titre in one inoculated pig remained low (no. 602). All contact pigs showed an increase in titre which is assumed to be due to continuation of the immune response against the vaccine virus Flc9.

After vaccination of the pigs in group B (Flc11), low percentages of CSFV-specific antibodies were detected with the E2-ELISA in some pigs (nos. 611, 615, 616-619). The other pigs remained negative (Table 3). In the E$^{RNS}$ ELISA, all pigs showed a low to intermediate inhibition percentage after vaccination (Table 4). The NPLA results were as follows: some vaccinated pigs (nos. 611, 614, 615-619) developed low titres of neutralising antibodies against CSFV (Table 5), but some pigs (nos. 611-613, 616-617) also developed low titres against BVDV strain 5250 (Table 6).

After challenge inoculation, a high inhibition percentage in the E2-ELISA was observed in all pigs (Table 3). Four of the inoculated pigs (nos. 611, 615, 617, 618) showed a positive inhibition percentage in the E$^{RNS}$-ELISA (Table 4). The fifth inoculated pig (620) showed a lower, intermediate inhibition percentage in the E$^{RNS}$-ELISA on the threshold of being positive. All contact pigs showed intermediate inhibition percentages in the E$^{RNS}$-ELISA at levels considered negative. In the neutralising antibody assays, all inoculated pigs developed high titres against CSFV (Table 5). None of the contact pigs showed high titres of neutralising antibodies against CSFV. Since neutralising antibodies are mainly directed against E2 of pestiviruses and strain 5250 is of a pestivirus type hardly cross-reacting serologically with CSFV, the results of the 5250 NPLA are low or negative for all animals in the Flc11 group.

## Transmission of virus

Based on all variables determined in the contact pigs (mentioned in the Material and Method section), all four contact pigs in the control group (C) became infected, and two contact exposed pigs in the Flc 9 group (A) became infected. None of the contact pigs in the Flc11 group (B) became infected. Based on these results, the following reproduction rates were calculated:

| | |
|---|---|
| $R_{control}$ | 10 |
| $R_{flc9}$ | 0.6 |
| $R_{flc11}$ | 0 |

## Description of the figures

**Figure 1.** Comparison of the deduced amino acid sequence of E$^{RNS}$ (A) and E2 (B) proteins of the indicated pestiviruses. The relevant genomic regions of BVDV strain 5250 were sequenced and then compared to those of the type I BVDV strains NADL and Osloss, Border disease virus strain BD31, type II BVDV strain 890 and CSFV strains Brescia and C. The positions of amino acid residues are based on those of CSFV strain C (Moormann *et al*., 1996. J. Virol.**70**:763-770; EMBL database nr. Z46258).

**Figure 2.** Schematic representation of the construction of pPRKflc6, pPRKflc7, pPRKflc9 and pPRKflc11. From the RNA genomes of BVDV strains 5250 and NADL fragments were amplified covering the E2 or E$^{RNS}$ genes through RT/PCR (indicated by arrows). These fragments were cloned in pGEM4z-bleu (not shown) and through PCR cloned in transient expression vectors resulting in pPRKh1, pPRKc13 and pPRKh18. Expression of BVDV strain 5250 E$^{RNS}$ and CSFV C-strain E2 genes in pPRKc13, and the strain 5250 E2 and C-strain E$^{RNS}$ genes in pPRKh18, was studied by transient expression in SK6 cells. Expression of BVDV strain NADL E2 and C-strain E$^{RNS}$ genes in pPRKh1, was studied by transient expression in SK6.T7 cells. Cloning of the *NcoI*/*XbaI* fragment from pPRc132 into pPRKh1 resulted in full-length clone pPRKflc6 (A). Cloning of the *ClaI*/*NgoMI* fragment from plasmid pPRKc13 into pPRKflc3 resulted in full-length clone pPRKflc7 (B). Cloning of the *ClaI*/*BglII* fragment of pPRKh18 into pPRKflc3 resulted in full-length clone pPRKflc9 (C). Cloning of the *ClaI*/*NgoMI* fragment of of pPRKc13 into pPRKflc2 resulted in full-length clone pPRKflc11 (D). N$^{PRO}$, autoprotease; C, core protein; E$^{RNS}$, E1 and E2, envelope proteins; NS2, NS23, NS3, NS4a, NS4b, NS5a, NS5b, non-structural genes; 5' NTR, 5' non-translated region; 3' NTR, 3' non-translated region; phCMV is the promotor-enhancer region of the immediate-early gene of human cytomegalovirus (hCMV); T7, bacteriophage T7 promotor sequence.

**Figure 3.** Multistep growthcurve of viruses Flc2, Flc9, Flc11 en 5250. Confluent monolayers of SK6 cells were infected with a multiplicity of infection of 0.1 with strains Flc2, Flc9, Flc11 and 5250. Virus titres were determined separately from supernatants and cell lysates. Virus titres (log TCID50 per millilitre) were established at 0, 6, 24, 48, 72, 96, 120 and 144 hours after infection.

Table 1

| Characterization of chimeric CSFV/BVDV recombinant C-strain viruses Reaction of CSFV and BVDV specific Mabs | | | | | | |
|---|---|---|---|---|---|---|
| strain | Directed against E2 | | | | Directed against $E^{RNS}$ | |
| | BVDV specific epitope[a] | BVDV specific epitope[b] | C-strain specific epitope[c] | CSFV specific epitope[d] | BVDV specific epitope[e] | C-strain specific epitope[f] |
| FLc2 | - | - | + | - | - | + |
| FLc6 | + | + | - | - | - | + |
| FLc7 | - | - | - | + | + | - |
| FLc9 | - | + | - | - | - | + |
| FLc11 | - | - | + | - | + | - |
| NADL | + | + | - | - | - | - |
| 5250 | - | + | - | - | + | - |
| Brescia | - | - | - | + | - | - |

a) Mab CT6 specifically recognizes an epitope on E2 of BVDV (Bolin *et al*., Arch. Virol. **99**, 117-123, 1988).

b) Mab Wb166 specifically recognizes an epitope on E2 of BVDV different from Mab CT6 (Paton *et al*., Virology **190**, 763-772, 1992).

c) Mab C2 specifically recognizes an epitope on E2 of CSFV strain C (Wensvoort, G., Ph. D thesis. State University of Utrecht, Utrecht, The Netherlands 1989).

d) Mab b6 recognizes an epitope in domain B of E2 of CSFV strain Brescia (Wensvoort *et al*., J. Gen. Virol. **70**, 2865-2876, 1989).

e) Mab Wb433 specifically recognizes an epitope on $E^{RNS}$ of BVDV (Paton *et al*., Arch. Virol. **135**, 241-252, 1994).

f) Mab C5 specifically recognizes an epitope on $E^{RNS}$ of CSFV strain C. (Wensvoort, G., Ph.D. thesis. State University of Utrecht, Utrecht, The Netherlands, 1989).

Animal experiment: 298-47042-00.22 / 97-11

Table 2

| Results of virus isolation, cytopenia and fever after challenge with CSFV | | | | | | | |
|---|---|---|---|---|---|---|---|
| group | pig no. | I/C | # days with fever[a] | viremia | IFT | cytopenia[b] | death |
| A. | 601 | c | 0 | - | - | - | - |
| Flc9 | 602 | i | 1 | - | - | - | - |
| | 603 | c | 0 | - | - | - | - |
| | 604 | c | 4 | - | - | - | - |
| | 605 | c | 1 | - | - | - | - |
| | 606 | i | 6 | - | - | - | - |
| | 607 | c | 4 | - | - | - | - |
| | 608 | i | 3 | - | - | - | - |
| | 609 | i | 6 | - | - | - | - |
| | 610 | i | 6 | - | - | - | - |
| | | | | | | | |
| B. | 611 | i | 0 | - | - | - | - |
| Flc11 | 612 | c | 0 | - | - | - | - |
| | 613 | c | 0 | - | - | - | - |
| | 614 | c | 0 | - | - | - | - |
| | 615 | i | 0 | - | - | - | - |
| | 616 | c | 0 | - | - | - | - |
| | 617 | i | 0 | - | - | - | - |
| | 618 | i | 0 | - | - | - | - |
| | 619 | c | 0 | - | - | - | - |
| | 620 | i | 0 | - | - | - | - |
| | | | | | | | |
| C. | 621 | i | 12 | + | + | + | + |
| contr | 622 | i | 12 | + | + | + | + |
| | 623 | c | 6 | + | + | + | + |
| | 624 | c | 7 | + | + | + | + |
| | 625 | i | 12 | + | + | + | + |
| | 626 | c | 6 | + | + | + | + |
| | 627 | c | 6 | + | + | + | + |

a) Fever: body temperature $\geq$ 40 °C
b) Cytopenia: thrombocytopenia and/or leucopenia
i: vaccinated and inoculated animal
c: vaccinated in contact animal
- : not detected/observed
+ : detected/observed

Animal experiment: 298-47042-00.22 / 97-11

Table 3

| Results of the Ceditest® ELISA for the detection of CSFV-E2 antibodies[a] | | | | | | | | | | | | | |
|---|---|---|---|---|---|---|---|---|---|---|---|---|---|
| Group | Animal no. | I/C | lab | days post challenge | | | | | | | | | |
| | | | | -26 | 0 | 14 | 15 | 16 | 21 | 28 | 35 | 42 | 49 |
| A. | 601 | c | 1 | 0 | 0 | 0 | | 19 | 78 | 90 | 94 | 99 | 100 |
| Flc9 | 602 | i | 2 | 0 | 0 | 71 | | | 100 | 102 | 102 | 102 | 102 |
| | 603 | c | 3 | 0 | 0 | 0 | | 0 | 6 | 53 | 90 | 87 | 93 |
| | 604 | c | 4 | 0 | 0 | 0 | | 0 | 0 | 0 | 0 | 0 | 0 |
| | 605 | c | 5 | 0 | 0 | 0 | | 0 | 0 | 0 | 0 | 0 | 0 |
| | 606 | i | 6 | 0 | 0 | 32 | | | 100 | 102 | 102 | 102 | 102 |
| | 607 | c | 7 | 0 | 0 | 0 | | 0 | 0 | 0 | 0 | 0 | 0 |
| | 608 | i | 8 | 0 | 0 | 72 | | | 101 | 102 | 102 | 102 | 102 |
| | 609 | i | 9 | 0 | 0 | 58 | | | 100 | 102 | 102 | 102 | 102 |
| | 610 | i | 10 | 0 | 0 | 95 | | | 102 | 102 | 102 | 102 | 102 |
| | | | | | | | | | | | | | |
| B. | 611 | i | 11 | 0 | 40 | 102 | | | 101 | 101 | 102 | 102 | 102 |
| Flc11 | 612 | c | 12 | 0 | 0 | 40 | | 62 | 84 | 81 | 82 | 89 | 93 |
| | 613 | c | 13 | 0 | 8 | 63 | | 34 | 79 | 85 | 78 | 85 | 88 |
| | 614 | c | 14 | 0 | 0 | 28 | | 56 | 80 | 83 | 78 | 90 | 95 |
| | 615 | i | 15 | 0 | 11 | 102 | | | 102 | 102 | 102 | 102 | 102 |
| | 616 | c | 16 | 0 | 20 | 34 | | 31 | 62 | 76 | 72 | 63 | 75 |
| | 617 | i | 17 | 0 | 35 | 103 | | | 102 | 102 | 102 | 102 | 102 |
| | 618 | i | 18 | 0 | 12 | 103 | | | 102 | 102 | 102 | 102 | 102 |
| | 619 | c | 19 | 0 | 14 | 45 | | 52 | 86 | 77 | 79 | 75 | 82 |
| | 620 | i | 20 | 0 | 0 | 103 | | | 102 | 102 | 102 | 102 | 102 |
| | | | | | | | | | | | | | |
| C. | 621 | i | 21 | 0 | 0 | 0 | 0 | | | | | | |
| contr | 622 | i | 22 | 0 | 0 | 0 | 0 | | | | | | |
| | 623 | c | 23 | 0 | 0 | 0 | 0 | | | | | | |
| | 624 | c | 24 | | 0 | 0 | 0 | | | | | | |
| | 625 | i | 25 | | 0 | 0 | 0 | | | | | | |
| | 626 | c | 26 | | 0 | 0 | 0 | | | | | | |
| | 627 | c | 27 | | 0 | 0 | 0 | | | | | | |

a) The Ceditest® E2-ELISA specifically detects antibodies against envelope protein E2 of CSFV. Test results are expressed as the percentage inhibition of a standard signal; <30% is negative , 30-50% inhibition is doubtful, >50% inhibition is positive.

Animal experiment: 298-47042-00.22 / 97-11

Table 4

| Results of the Ceditest® ELISA for the detection of CSFV-E[RNS] antibodies[a] | | | | | | | | | | | | | |
|---|---|---|---|---|---|---|---|---|---|---|---|---|---|
| Group | Animal no. | I/C | lab | days post challenge | | | | | | | | | |
| | | | | -26 | 0 | 14 | 15 | 16 | 21 | 28 | 35 | 42 | 49 |
| A. | 601 | c | 1 | 29 | 46 | 97 | | 99 | 98 | 99 | 100 | 101 | 100 |
| Flc9 | 602 | i | 2 | 24 | 38 | 0 | | | 8 | 17 | 53 | 58 | 62 |
| | 603 | c | 3 | 26 | 30 | 47 | | 55 | 51 | 47 | 52 | 46 | 48 |
| | 604 | c | 4 | 20 | 36 | 7 | | 39 | 33 | 33 | 33 | 45 | 42 |
| | 605 | c | 5 | 21 | 46 | 27 | | 35 | 44 | 57 | 61 | 66 | 68 |
| | 606 | i | 6 | 28 | 44 | 97 | | | 99 | 101 | 101 | 101 | 101 |
| | 607 | c | 7 | 20 | 41 | 64 | | 69 | 54 | 45 | 60 | 57 | 58 |
| | 608 | i | 8 | 21 | 30 | 93 | | | 93 | 93 | 94 | 95 | 94 |
| | 609 | i | 9 | 29 | 41 | 97 | | | 98 | 96 | 98 | 99 | 99 |
| | 610 | i | 10 | 20 | 15 | 102 | | | 102 | 102 | 102 | 102 | 102 |
| | | | | | | | | | | | | | |
| B. | 611 | i | 11 | 23 | 36 | 67 | | | 41 | 56 | 68 | 73 | 74 |
| Flc11 | 612 | c | 12 | 43 | 46 | 30 | | 57 | 29 | 0 | 16 | 10 | 44 |
| | 613 | c | 13 | 28 | 13 | 21 | | 45 | 0 | 0 | 0 | 0 | 19 |
| | 614 | c | 14 | 33 | 22 | 5 | | 51 | 9 | 1 | 26 | 18 | 21 |
| | 615 | i | 15 | 35 | 29 | 2 | | | 61 | 72 | 80 | 79 | 77 |
| | 616 | c | 16 | 21 | 28 | 0 | | 23 | 11 | 17 | 17 | 31 | 17 |
| | 617 | i | 17 | 50 | 17 | 6 | | | 21 | 43 | 48 | 54 | 64 |
| | 618 | i | 18 | 38 | 0 | 0 | | | 15 | 43 | 67 | 65 | 70 |
| | 619 | c | 19 | 18 | 0 | 49 | | 7 | 0 | 3 | 18 | 0 | 29 |
| | 620 | i | 20 | 19 | 0 | 0 | | | 0 | 35 | 58 | 39 | 49 |
| | | | | | | | | | | | | | |
| C. | 621 | i | 21 | 21 | 30 | 52 | 45 | | | | | | |
| contr | 622 | i | 22 | 14 | 28 | 45 | 40 | | | | | | |
| | 623 | c | 23 | 46 | 31 | 32 | 47 | | | | | | |
| | 624 | c | 24 | | 27 | 33 | 50 | | | | | | |
| | 625 | i | 25 | | 38 | 31 | 53 | | | | | | |
| | 626 | c | 26 | | 34 | 22 | 41 | | | | | | |
| | 627 | c | 27 | | 27 | 21 | 40 | | | | | | |

a) The Ceditest® E[RNS]-ELISA specifically detects antibodies against envelope protein E[RNS] of CSFV. The test results are expressed as the percentage inhibition of a standard signal; <50% is negative, ≥ 50% is positive.

Animal experiment: 298-47042-00.22 / 97-11

Table 5

| | | | | days post challenge | | | | | | | | | |
|---|---|---|---|---|---|---|---|---|---|---|---|---|---|
| Group | Ani-mal no. | I/C | lab | -26 | 0 | 14 | 15 | 16 | 21 | 28 | 35 | 42 | 49 |
| A. | 601 | c | 1 | <12,5 | <12,5 | 100 | | 200 | 400 | 400 | 1600 | >1600 | >1600 |
| Flc9 | 602 | i | 2 | <12,5 | <12,5 | 100 | | | 400 | 600 | >1600 | 1600 | >1600 |
| | 603 | c | 3 | <12,5 | <12,5 | 25 | | 19 | 100 | 100 | 600 | 200 | 400 |
| | 604 | c | 4 | <12,5 | <12,5 | <12,5 | | <12,5 | 50 | 25 | 75 | 12,5 | <12,5 |
| | 605 | c | 5 | <12,5 | <12,5 | <12,5 | | <12,5 | 37 | <12,5 | 37 | <12,5 | 12,5 |
| | 606 | i | 6 | <12,5 | <12,.5 | 150 | | | 600 | 1600 | 1600 | >1600 | >1600 |
| | 607 | c | 7 | <12,5 | <12,5 | 150 | | <12,5 | 25 | <12,5 | 300 | 75 | 50 |
| | 608 | i | 8 | <12,5 | <12,5 | 400 | | | >1600 | >1600 | >1600 | >1600 | >1600 |
| | 609 | i | 9 | <12,5 | <12,5 | 800 | | | 1600 | >1600 | >1600 | >1600 | >1600 |
| | 610 | i | 10 | <12,5 | <12,5 | 800 | | | 800 | >1600 | >1600 | >1600 | >1600 |
| | | | | | | | | | | | | | |
| B. | 611 | i | 11 | 12,5 | 37 | 1600 | | | >1600 | >1600 | >1600 | >1600 | >1600 |
| Flc11 | 612 | c | 12 | <12.5 | <12,5 | 150 | | 150 | 150 | 200 | 600 | 300 | 400 |
| | 613 | c | 13 | <12.5 | <12,5 | 75 | | 50 | 37 | 75 | 75 | 100 | 150 |
| | 614 | c | 14 | <12.5 | 12,5 | 50 | | 50 | 150 | 400 | 400 | 400 | 800 |
| | 615 | i | 15 | <12.5 | 12,5 | >1600 | | | >1600 | >1600 | >1600 | >1600 | 1600 |
| | 616 | c | 16 | <12.5 | 19 | 75 | | 150 | 50 | 100 | 150 | 50 | 100 |
| | 617 | i | 17 | 19 | 19 | >1600 | | | >1600 | >1600 | >1600 | >1600 | 1600 |
| | 618 | i | 18 | <12.5 | 12,5 | >1600 | | | >1600 | >1600 | >1600 | >1600 | >1600 |
| | 619 | c | 19 | <12.5 | 12,5 | 75 | | 100 | 200 | 300 | 400 | 150 | 150 |
| | 620 | i | 20 | <12.5 | <12,5 | >1600 | | | 1200 | >1600 | >1600 | 1600 | >1600 |
| | | | | | | | | | | | | | |
| C. | 621 | i | 21 | <12,5 | <12,5 | <12,5 | <12,5 | | | | | | |
| contr | 622 | i | 22 | <12.5 | <12,5 | <12,5 | <12,5 | | | | | | |
| | 623 | c | 23 | <12,5 | <12,5 | <12,5 | <12,5 | | | | | | |
| | 624 | c | 24 | | mv | <12,5 | 200 | | | | | | |
| | 625 | i | 25 | | mv | <12,5 | <12,5 | | | | | | |
| | 626 | c | 26 | | mv | <12,5 | <12,5 | | | | | | |
| | 627 | c | 27 | | 12,5 | <12,5 | <12,5 | | | | | | |

mv: missing value

Animal experiment: 298-47042-00.22 / 97-11

Table 6

| Results of the NPLA for the detection of BVDV 5250-specific neutralizing antibodies | | | | | | | | | | | | | | |
|---|---|---|---|---|---|---|---|---|---|---|---|---|---|---|
| Group | Animal no. | I/C | lab | days post challenge | | | | | | | | | | |
| | | | | -26 | 0 | 14 | 15 | 16 | 21 | 28 | 35 | 42 | 49 |
| A. | 601 | c | 1 | <12,5 | <12,5 | 75 | | 200 | 300 | 300 | 200 | 600 | 200 |
| Flc9 | 602 | i | 2 | <12,5 | 25 | 50 | | | 100 | 37 | 75 | 37 | 25 |
| | 603 | c | 3 | <12,5 | 37 | 75 | | 400 | 600 | 400 | 600 | 200 | 200 |
| | 604 | c | 4 | <12,5 | 12,5 | 100 | | 50 | 50 | 100 | 150 | 100 | 37 |
| | 605 | c | 5 | <12,5 | <12,5 | 50 | | 25 | 50 | 75 | 200 | 100 | 100 |
| | 606 | i | 6 | <12,5 | <12,.5 | 100 | | | 200 | 400 | 300 | 200 | 300 |
| | 607 | c | 7 | <12,5 | 37 | 100 | | 100 | 200 | 100 | 75 | 75 | 100 |
| | 608 | i | 8 | <12,5 | 19 | 800 | | | 600 | 800 | 300 | 200 | 200 |
| | 609 | i | 9 | <12,5 | <12,5 | 1600 | | | >1600 | 800 | 600 | 1200 | 800 |
| | 610 | i | 10 | <12,5 | <12,5 | 800 | | | 800 | 1200 | 600 | 300 | 300 |
| | | | | | | | | | | | | | |
| B. | 611 | i | 11 | 19 | 12,5 | 100 | | | 50 | 100 | 75 | 25 | 37 |
| Flc11 | 612 | c | 12 | <12.5 | 37 | 37 | | 75 | 75 | 50 | 25 | 37 | 75 |
| | 613 | c | 13 | <12.5 | 12,5 | 37 | | 50 | 19 | 19 | 37 | 19 | <12,5 |
| | 614 | c | 14 | <12.5 | <12,5 | <12,5 | | <12,50 | 12,5 | 25 | <12,5 | <12,5 | <12,5 |
| | 615 | i | 15 | <12,5 | <12,5 | 12,5 | | | 75 | 50 | 25 | <12,5 | <12,5 |
| | 616 | c | 16 | 12,5 | 19 | <12,5 | | 12,5 | <12,5 | <12,5 | 12,5 | <12,5 | <12,5 |
| | 617 | i | 17 | 19 | 37 | 37 | | | 150 | 50 | 50 | 25 | 25 |
| | 618 | i | 18 | mv | <12,5 | 150 | | | 400 | 100 | 37 | 50 | 25 |
| | 619 | c | 19 | <12.5 | <12,5 | 25 | | 19 | 75 | 37 | 19 | <12,5 | 37 |
| | 620 | i | 20 | <12.5 | <12,5 | 50 | | | 75 | 100 | 150 | 50 | <12,5 |
| | | | | | | | | | | | | | |
| C. | 621 | i | 21 | <12,5 | <12,5 | <12,5 | <12,5 | | | | | | |
| contr | 622 | i | 22 | <12,5 | 12,5 | <12,5 | <12,5 | | | | | | |
| | 623 | c | 23 | <12,5 | mv | <12,5 | <12,5 | | | | | | |
| | 624 | c | 24 | | mv | <12,5 | <12,5 | | | | | | |
| | 625 | i | 25 | | mv | <12,5 | <12,5 | | | | | | |
| | 626 | c | 26 | | mv | <12,5 | <12,5 | | | | | | |
| | 627 | c | 27 | | <12,5 | <12,5 | <12,5 | | | | | | |

mv: missing value

# EP 0 982 402 A1

## Claims

1. A recombinant nucleic acid derived from a genotype of a pestivirus wherein a first fragment or part thereof encoding at least one immunodominant part at least partly responsible for providing protection against a wild-type pestivirus infection of a viral protein has been modified to render said immunodominant part incapable of eliciting antibodies specific for said genotype.

2. A recombinant nucleic acid derived from a genotype of a pestivirus wherein a first fragment or part thereof encoding at least one immunodominant part at least partly responsible for providing protection against a wild-type pestivirus infection of a viral protein has been replaced by a second fragment or part thereof encoding an antigenically unrelated part of a protein from another genotype pestivirus.

3. A nucleic acid according to claim 1 or 2 wherein said viral protein is an E2 protein.

4. A nucleic acid according to claim 3 wherein said first fragment comprises a nucleic acid encoding E2 protein running from about amino acid position 690 to 865 or 692 to 877 or 690 to 1008.

5. A nucleic acid according to claim 2 wherein said protein from another genotype pestivirus is an E2 protein.

6. A nucleic acid according to claim 5 wherein said second fragment comprises a nucleic acid encoding E2 protein running from about amino acid position 690 to 865 or 692 to 877 or 690 to 1008.

7. A nucleic acid according to claim 1 or 2 wherein said viral protein is an E$^{rns}$ protein.

8. A nucleic acid according to claim 7 wherein said first fragment comprises a nucleic acid fragment encoding E$^{RNS}$ protein running from about amino acid position 268 to 494.

9. A nucleic acid according to claim 2 wherein said protein from another genotype pestivirus is an E$^{rns}$ protein.

10. A nucleic acid according to claim 9 wherein said second fragment comprises a nucleic acid encoding E$^{RNS}$ protein running from about amino acid position 268 to 494.

**11.** A chimeric pestivirus comprising a nucleic acid according to anyone of claims 1 to 10.

**12.** A chimeric pestivirus according to claim 11 wherein said chimeric pestivirus is deposited as I-2058 at 31 July 1998 at the CNCM, Paris, France.

**13.** A chimeric pestivirus according to claim 11 wherein said chimeric pestivirus is deposited as I-2059 at 31 July 1998 at the CNCM, Paris, France.

**14.** A chimeric pestivirus according to claim 11 wherein said chimeric pestivirus is deposited as I-2060 at 31 July 1998 at the CNCM, Paris, France.

**15.** A chimeric pestivirus according to claim 11 wherein said chimeric pestivirus is deposited as I-2061 at 31 July 1998 at the CNCM, Paris, France.

**16.** A vaccine comprising a chimeric pestivirus according to anyone of claims 11 to 15 allowing serological discrimination of an animal vaccinated with said vaccine from an animal infected with wild-type pestivirus.

**17.** A vaccine according to claim 16 capable of providing sufficient herd immunity to a vaccinated population to reduce the reproduction ratio ($R_0$) of said wild-type pestivirus to at least below 1.

**18.** A vaccine according to claim 16 or 17 wherein said chimeric pestivirus is derived from a pestivirus vaccine strain, such as C-strain virus.

**19.** A vaccine according to anyone of claims 16 to 18 wherein said wild-type pestivirus is a classical swine fever virus.

**20.** A vaccine according to anyone of claims 16 to 18 wherein said wild-type pestivirus is a bovine viral diarrhoea virus.

**21.** A vaccine according to anyone of claims 16 to 18 wherein said wild-type pestivirus is a Border disease virus.

**22.** A method for controlling and/or eradicating a wild-type pestivirus infection comprising vaccinating at least a part of an animal population with a vaccine according to anyone of claims 16 to 22 and further comprising testing at least a part of said population for the presence of antibodies specific for said wild-type pestivirus.

# Figure 1A

```
NADL              ENITQWNLQDNGTEGIQRAMFQRGVNRSLHGIWPEKICTGVPSHLATDIELKTIHGMMDA
Osloss            """"""""""""""""""""""""""""""""""""""""""""""T"""A"""""""
BD31              """"""""S""""H"""H""""""""""""""""""""""""""T"""""T""RG"Q"""""
890               """""""M"""""""""Q"""L""""""""""""""""""""""T"""""""Y""RE"V"""""
5250              """""""M"":""""""""""L""""""""""""""""""""I"T"""""""Y"""E"V"""""
C-strain   268    """"""""S""""""N"""H""YL""""""""""""""""""""K"""T"""""V"""E"Q"""""


NADL              SEKTNYTCCRLQRHEWNKHGWCNWYNIEPWILVMNRTQANLTEGQPPRECAVTCRYDRAS
Osloss            """""""""""""""""""""""""""""""VL""K"""""A"""""""""""""""""D"
BD31              """""""""""""""""""""""""""""N"""W"""K"""""""P"EK"T""""F"KE"
890               """""""""""""""""""""""""FH"""""WL""K"N""""""""K""""""""""KEA
5250              """""""""""""""""""""""""FH"""""WL""K"S"""""""L""""""""""""ET
C-strain          ""G"""""""K""""""""""""""H""D"""QL"""""D"A""P"VK"""""""""""KDA


NADL              DLNVVTQARDSPTPLTGCKKGKNFSFAGILMRGPCNFEIAASDVLFKEHERISMFQDTTL
Osloss            """"""""""N"""""""""""""""""V"VQ""""""""V""""""R""DCT"VI"G"AH
BD31              "M"""""""R""T"""""""""K""""""MIIE"""""NVSVE"I""GDS"CS"L""""A"
890               E""I"""""R""T""""""""""""""VILD"""""KVSVE"""""""DCGN"L"E"AI
5250              E""I"""""R""T""""""""""""""VLN"""""KVSVEE"""""""DCGN"L"E"AI
C-strain          "I"""""""NR""T""""""""""""""TVIES"""""NVSVE"T"YGD""CG"LL""AA"


NADL              YLVDGLTNSLEGARQGTAKLTTWLGKQLGILGKKLENKSKTWFGAYA
Osloss            """"""M"""""S"""""""""""""R""KK""""""""""""""""
BD31              "V"""V""TV"S""""A""""S"""""""""M"""""H"""""""""N"
890               Q"L""A""TI""""V"""""""""""""""""""""""T"A""""H"
5250              Q"L""A""TI""""V"""""""""""""""R"""R""""""""S"""H"
C-strain          """"""M""TI"N"""""A"RV"S"""R""RTA""R""GR"""""""""  494
```

# Figure 1B

```
NADL              HLDCKPEFSYAIAKDERIGQLGAEGLTTTWKEYSPGMKLEDTMVIAWCEDGKLMYLQRCT
Osloss            LPV"""G"Y"""""NNE""P"""T"""Q"Y"""D""R"Q""G"VV""KG"EIK""IT"E
BD31              EFA"REDHR""L""TKE""P"""""S"""""TD"QRSFD"D"GA"R"S"VG"YFRFREH"L
890               FPE""EG"Q"""S""RKM"L""P"S"""""HRPT__K""V"S""QV"""GKD"KI"KT"P
5250              FPE""EG"Q"""S""KK"L""P"S"""""HLPT__K""V"S""QV"""GKD"KI"KT""
Brescia           R"A""EDHR"""STTNE""LH"""""""""""NHNLQ"D"GT"K"I"MA"SFKVTALNV
C-strain      690 R"A""EDYR"""SSTDE""L"""G."""""""""NHDLQ"N"GT"K"S"VA"SFKVTALNV


NADL              RETRYLAILHTRALPTSVVFKKLFDGRKQEDVVEMNDNFEFGLCPCDAKPIVRGKFNTTL
Osloss            ""A"""""""""""""""""E"II""KE"""""""D""""L"""""""""L"""""""""
BD31              IGS"""""S""Q"""""""T"ELIPG"STLVEE_""G"D"""""""""SR"L"""""Y"A""
890               K"E"""VAV"E"""S""AE"MPIS""TIGP""ID"P"D"""""""""""VIK"""""AS"
5250              ""E"""VAV"E"""S""AE"MQIS""KLGPS"ID"P"D"""""""""""S""VIK"""""AS"
Brescia           VSR"""""S""KD"""""""T"EL"""""TSPLTE_""G"D"G"""""F"TS"V"K""F"""""
C-strain          VSR"""""S""KK"""""""T"EL"""""TNPSTE_""G"D"RS"""""F"TS"V"K""Y"""""


NADL              LNGPAFQMVCPIGWTGTVS_CTSFNMDTLATTVVRTYRRSKPFPHRQGCITQKNLGED
Osloss            """""""""""""""""""L"HWS"K"""M""""""K"HR"""F""""""""VI"G"
BD31              """S"""L"""YE"""R"E_""TISKS""""E""KI"K"T"R"RSGLVATHTTIYE""
890               """"""""""""Q"""""IE_""LA"Q"""D"""""""""TT""QR"KW"SYE"II"""
5250              """""""""""Q"""RIE_"""V"Q"""D"""""""""TT""QR"RW"VYE"MI"""
Brescia           """S""YL"""""""VIE_""AVSPT""R"E""K"F""E""""Y"RD"V"TTVEN""
C-strain          """S""YL"""""""VIE_""AVSPT""R"E""K"F""D"""""MD"V"TTVEN""  865
```

# Figure 2A

RNA genome of BVDV strain NADL

RT/PCR

pPRKh1

confirmation of strain specific
expression of E$^{RNS}$ and E2

NcoI/XbaI from pPRc132
in pPRKh1

pPRc132

pPRKflc6

# Figure 2B

RNA genome of BVDV strain 5250

# Figure 2C

RNA genome of BVDV strain 5250

RT/PCR

pPRKh18

confirmation of strain specific
expression of E$^{RNS}$ and E2

ClaI/BglII in pPRKflc3

pPRKflc3

pPRKflc9

# Figure 2D

Figure 3

EP 0 982 402 A1

## European Patent Office

## PARTIAL EUROPEAN SEARCH REPORT

which under Rule 45 of the European Patent Convention shall be considered, for the purposes of subsequent proceedings, as the European search report

**Application Number**

EP 98 20 2737

### DOCUMENTS CONSIDERED TO BE RELEVANT

| Category | Citation of document with indication, where appropriate. of relevant passages | Relevant to claim | CLASSIFICATION OF THE APPLICATION (Int.Cl.6) |
|---|---|---|---|
| D,X | R.J.M. MOORMANN ET AL. : "Infectious RNA transcribed from am engineered full-length cDNA template of the genome of a Pestivirus" JOURNAL OF VIROLOGY., vol. 70, no. 2, February 1996, pages 763-770, XP002089686 | 1-3,5,7, 9,11-22 | C12N15/40 C07K14/08 C12N7/01 A61K39/12 |
| Y | * page 769, right-hand column, paragraph 2 * <br> * page 767, right-hand column, paragraph 3 - page 768, left-hand column, paragraph 5 * <br> * page 763, right-hand column, paragraph 3 - page 764, left-hand column, paragraph 1 * <br> * abstract * <br> --- <br> -/-- | 4,6 | |

**TECHNICAL FIELDS SEARCHED (Int.Cl.6)**

C12N
C07K
A61K

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

Although claim 22 is directed to a method of treatment of the animal body (Article 52(4) EPC), the search has been carried out and based on the alleged effects of the composition.

| Place of search | Date of completion of the search | Examiner |
|---|---|---|
| THE HAGUE | 2 March 1999 | Montero Lopez, B |

## PARTIAL EUROPEAN SEARCH REPORT

**European Patent Office**

**Application Number**

EP 98 20 2737

### DOCUMENTS CONSIDERED TO BE RELEVANT

| Category | Citation of document with indication, where appropriate, of relevant passages | Relevant to claim |
|---|---|---|
| X<br>Y | WO 95 35380 A (INSTITUUT VOOR VEEHOUDERIJ EN DIERGEZONDHEID) 28 December 1995<br><br>* page 2, line 24 - page 3, line 23 *<br>* page 3, line 29 - page 4, line 12 *<br>* page 6, line 11 - page 8, line 27 *<br>* page 9, line 30 - page 10, line 14 *<br>* page 13, line 3 - line 11 *<br>* page 13, line 26 - page 14, line 32; example 5 * | 1-3,5,<br>11-22<br>4,6 |
| D,Y | P.A. VAN RIJN ET AL.: "Subdivision of the Pestivirus genus based on envelope glycoprotein E2"<br>VIROLOGY,<br>vol. 237, no. 2, 27 October 1997, pages 337-348, XP002089687<br>ORLANDO US<br>* page 338, left-hand column, paragraph 2 *<br>* page 341, left-hand column, paragraph 2 - page 344, left-hand column, paragraph 1 * | 4,6 |
| D,A | R.J.M. MOORMANN ET AL.: "Recent developments in pig vaccination"<br>PROCEEDINGS OF THE 14TH IPVS CONGRESS, 7 - 10 July 1996, pages 25-29, XP002089688<br>Bologna, Italy<br>* the whole document * | 1-22 |

**CLASSIFICATION OF THE APPLICATION (Int.Cl.6)**

**TECHNICAL FIELDS SEARCHED (Int.Cl.6)**

EPO FORM 1503 03.82 (P04C10)

## ANNEX TO THE EUROPEAN SEARCH REPORT
## ON EUROPEAN PATENT APPLICATION NO.

EP 98 20 2737

This annex lists the patent family members relating to the patent documents cited in the above-mentioned European search report.
The members are as contained in the European Patent Office EDP file on
The European Patent Office is in no way liable for these particulars which are merely given for the purpose of information.

02-03-1999

| Patent document cited in search report | Publication date | Patent family member(s) | Publication date |
|---|---|---|---|
| WO 9535380 A | 28-12-1995 | AU 697866 B | 22-10-1998 |
| | | AU 2631595 A | 15-01-1996 |
| | | BR 9508054 A | 12-08-1997 |
| | | CA 2192940 A | 28-12-1995 |
| | | CN 1151185 A | 04-06-1997 |
| | | CZ 9603696 A | 11-06-1997 |
| | | EP 0766740 A | 09-04-1997 |
| | | HU 76352 A | 28-08-1997 |
| | | JP 9511914 T | 02-12-1997 |
| | | NZ 287563 A | 26-08-1998 |
| | | PL 317755 A | 28-04-1997 |
| | | ZA 9505042 A | 08-02-1996 |

EPO FORM P0459

For more details about this annex : see Official Journal of the European Patent Office, No. 12/82